# FASCICULE DE BREVET EUROPEEN

(11) **EP 4 454 768 B1**
(45) Date de publication et mention de la délivrance du brevet: **06.08.2025**
(21) Numéro de dépôt: 24169281.3
(22) Date de dépôt: 09.04.2024
(51) Int. Cl.: B06B 1/06

(54) **DISPOSITIF DE TRANSDUCTION ULTRASONORE A ADRESSAGE LIGNE-COLONNE**
ULTRASCHALLWANDLER MIT ZEILEN-SÄULEN-ADRESSIERUNG
ROW-COLUMN-ADDRESSED ULTRASONIC TRANSDUCTION DEVICE

(30) Priorité: 24.04.2023 FR 2304069
(43) Date de publication de la demande: 30.10.2024
(73) Titulaire: Vermon, 37000 Tours (FR)
(72) Inventeur: LEGROS, Mathieu, 37000 Tours (FR); LAFON, Stéphane, 37000 Tours (FR)
(74) Mandataire: Cabinet Beaumont

(56) Documents cités:
- US-A- 4 550 606
- US-A1- 2019 328 360
- US-A1- 2021 302 388

## Description

### Domaine technique

La présente description concerne des dispositifs de transduction ultrasonore comprenant une matrice de transducteurs ultrasonores élémentaires connectés électriquement par des réseaux d'électrodes en ligne et en colonne appelés dispositifs de transduction ultrasonores à adressage ligne-colonne ou dispositifs de transduction ultrasonores RCA (de l'anglais "Row Column Addressing").

Les documents US2019/328360, US2021/302388 et US4550606 décrivent des exemples de dispositifs de transduction ultrasonore. Le document US2019/328360 divulgue un transducteur à ultrasons comprenant une première couche piézoélectrique empilée sur une seconde couche piézoélectrique pour former un empilement. La première couche piézoélectrique comporte une face principale métallisée pour former un premier réseau d'électrodes et l'autre face principale métallisée pour former une première électrode de masse. La seconde couche piézoélectrique comporte une face principale métallisée pour former un second réseau d'électrodes et l'autre face principale métallisée pour former une seconde électrode de masse, le second réseau d'électrodes étant orienté selon un angle par rapport au premier réseau d'électrodes.

### Technique antérieure

Des dispositifs de transduction ultrasonore à adressage ligne-colonne sont devenus de plus en plus attractifs dans le domaine de l'imagerie ultrasonore car ils permettent une imagerie simultanée dans au moins deux plans, et peuvent également être utilisés pour une imagerie en volume 3D.

L'un des avantages principaux des transducteurs RCA réside dans le nombre de circuits électroniques réduit nécessaires pour piloter les éléments du transducteur. Pour une matrice composée de R lignes et C colonnes, il faut typiquement R+C canaux de pilotage dans le cas d'une matrice RCA alors que typiquement RxC canaux sont nécessaires pour une matrice entièrement peuplée, autrement dit pour laquelle chaque transducteur élémentaire est connecté individuellement. Dans une application dans laquelle R = C, un système de pilotage électronique possédant typiquement 64, 128 ou 256 canaux peut piloter un dispositif RCA dont le nombre d'éléments est respectivement de 32x32, 64x64 ou 128x128, alors que pour un dispositif de transduction ultrasonore entièrement peuplé, le nombre maximum de transducteurs élémentaires sera respectivement limité à 8x8, 11x11 ou 16x16.

Pour les applications dans lesquelles les circuits électroniques de pilotage des transducteurs sont déportés de la sonde comprenant la matrice de transducteurs ultrasonores, une matrice RCA permet avantageusement de réduire le nombre de câbles coaxiaux entre la sonde et le système électronique déporté. Alternativement, la réduction du nombre de canaux permet avantageusement d'intégrer les circuits électroniques de pilotage dans la sonde au plus près de la matrice de transducteurs élémentaires. Pour des applications dans lesquelles des interconnexions de type carte de circuit imprimé (PCB - de l'anglais "Printed Circuit Board") ou carte de circuit imprimé souple (flex) sont utilisées pour connecter les transducteurs aux câbles coaxiaux ou à des circuits électroniques de pilotage embarqués au plus près des transducteurs, les transducteurs RCA réduisent avantageusement le nombre d'interconnexions et permettent par conséquent d'avoir des PCB moins complexes en diminuant le nombre de couches de PCB nécessaires ainsi que le nombre de connexions entre couches (vias). Des PCB plus minces réduisent l'impact sur la propagation acoustique, mais, comme cela sera discuté ultérieurement, la désadaptation d'impédance causée par les matériaux des PCB reste malgré tout un problème.

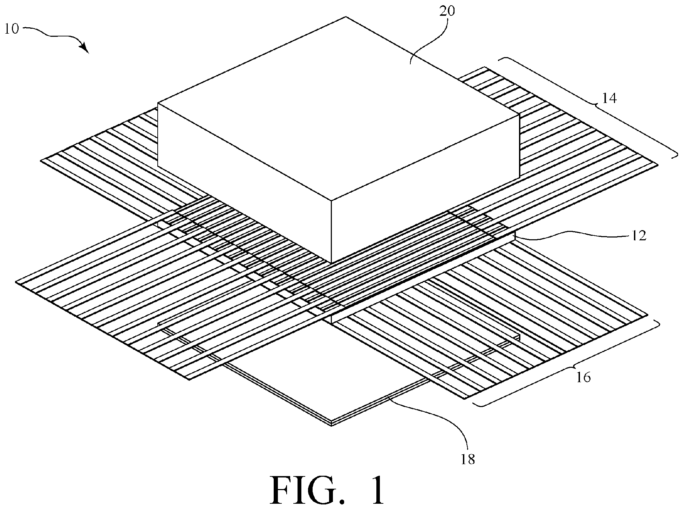

La figure 1 illustre un exemple d'un transducteur RCA 10, comportant une couche de matériau actif 12 (par exemple, un matériau piézoélectrique, piézocomposite, ou monocristallin), un réseau d'interconnexion de ligne 14 (R), un réseau d'interconnexion de colonne 16 (C), une couche d'adaptation d'impédance acoustique 18, et une couche de face arrière 20. Les surfaces principales du matériau actif 12 sont entièrement ou partiellement métallisées dans les zones en vis-à-vis des réseaux de colonne 16 et de ligne 14 (non représenté sur la figure). La couche d'adaptation d'impédance acoustique 18 est une couche ou des couches de matériau ayant une impédance acoustique dont la valeur se situe entre l'impédance acoustique de la couche de matériau actif 12 et l'impédance acoustique d'un objet en train d'être imagé (par exemple, un corps humain) pour optimiser la transmission de l'onde acoustique entre les deux milieux. La surface extérieure principale de la couche d'adaptation d'impédance constitue donc la face de sortie des ondes acoustiques émises par le transducteur. La couche de face arrière 20 est une couche ou un multicouches d'un ou plusieurs matériaux acoustiquement absorbant pour éviter des réflexions parasites d'ondes acoustiques. Par convention, le réseau d'interconnexion de ligne 14 (R) se trouve entre la couche de matériau actif 12 et la couche de matériau de face arrière 20, et le réseau d'interconnexion de colonne 16 (C) se trouve entre la couche de matériau actif 12 et la couche de matériau acoustique 18.

Toutefois, la présence de matériaux d'interconnexion, comprenant par exemple des matériaux électriquement isolants tels que du polyimide et des matériaux électriquement conducteurs tels que le cuivre, peut affecter négativement les performances du dispositif de transduction ultrasonore RCA 10, comme cela sera discuté ultérieurement, et la fabrication/l'assemblage de la structure d'interconnexion classique est compliqué du fait que les deux réseaux d'électrodes (électrodes de réseau R & électrodes de réseau C) doivent être interconnectées au niveau de surfaces (faces) principales opposées de la couche de matériau actif 12.

S'agissant des matériaux d'interconnexion, le dispositif de transduction ultrasonore RCA 10 requiert au moins le réseau d'interconnexion 16 (C) entre la couche de matériau actif 12 et la couche d'adaptation d'impédance acoustique 18. Certains désavantages du réseau d'interconnexion 16 (C) incluent une désadaptation d'impédance entre la couche de matériau actif 12, le réseau d'interconnexion 16 (C) et la couche d'adaptation d'impédance acoustique 18, et une faible uniformité entre les éléments. S'agissant de la désadaptation d'impédance, le réseau d'interconnexion 16 (C) comprend un matériau isolant de faible impédance acoustique, typiquement de 3 MRayl, par rapport à la couche de matériau actif 12, typiquement de 30 MRayl, et, par conséquent, crée un désaccord d'impédance acoustique important entre la couche de matériau actif 12 et la couche d'adaptation acoustique 18. Une solution possible pourrait être que la couche d'interconnexion s'étende seulement partiellement au-dessus des surfaces métallisées du réseau de colonnes. Typiquement la couche d'interconnexion recouvre une (ou les deux) extrémité(s) des extensions de surfaces métallisées du réseau de colonne et s'étend le long de ces surfaces métallisées au-dessus de quelques éléments latéraux du réseau de ligne. Dans une telle configuration, la hauteur de la couche d'interconnexion crée un espace entre les surfaces métallisées du réseau de colonne et la couche de matériau acoustique 12. Même si l'espace peut être rempli avec un matériau, s'agissant de l'uniformité, les propriétés acoustiques ne sont par conséquent pas identiques sur toute la surface du transducteur, en fonction de la longueur de l'extension de la couche d'interconnexion sur les surfaces métallisées du réseau de colonne, la charge acoustique, c'est-à-dire l'impédance vue par une partie des éléments, n'est pas la même. S'agissant de la qualité de la propagation acoustique, il est toujours souhaitable d'avoir un contact direct entre la couche de matériau actif 12 et la couche d'adaptation acoustique 18, ou à défaut d'avoir un matériau adhésif interposé entre elles aussi mince que possible.

### Résumé de l'invention

Un mode de réalisation prévoit un dispositif de transduction ultrasonore matriciel à adressage ligne-colonne, comprenant :
- une plaque de matériau actif métallisée comportant une plaque de matériau actif ayant une première surface principale et une deuxième surface principale à l'opposé de la première surface principale, une première couche métallique de surface principale sur la première surface principale, et une deuxième couche métallique de surface principale sur la deuxième surface principale ;
- une couche d'adaptation d'impédance acoustique fixée à la deuxième couche métallique de surface principale ;
- un premier ensemble d'encoches de découpe parallèles orientées dans une première direction, lesdites encoches du premier ensemble s'étendant à travers toute l'épaisseur de la plaque de matériau actif métallisée et sur toute la longueur de la plaque de matériau actif métallisée dans la première direction, et individualisant la deuxième couche métallique de surface principale en un ensemble d'électrodes de colonne ;
- un deuxième ensemble d'encoches de découpe parallèles orientées selon une deuxième direction différente de la première direction, lesdites encoches du deuxième ensemble s'étendant à travers toute l'épaisseur de la première couche métallique de surface principale et à travers au moins une partie de l'épaisseur de la plaque de matériau actif, et sur toute la longueur de la plaque de matériau actif métallisée dans la deuxième direction, et individualisant la première couche métallique de surface principale en un ensemble de lignes d'électrodes de ligne et une première ligne externe de contacts d'électrode de colonne, chaque contact d'électrode de colonne de la première ligne externe de contacts d'électrode de colonne étant connecté électriquement à une électrode de colonne respective de l'ensemble d'électrodes de colonne ; et
- une couche d'interconnexion comportant un substrat d'un matériau isolant électriquement, un premier ensemble de pistes conductrices sur le substrat, chaque piste du premier ensemble de pistes conductrices étant en contact électrique avec un contact d'électrode de colonne respectif de la première ligne externe de contacts d'électrode de colonne, et un deuxième ensemble de pistes conductrices sur le substrat, chaque piste du deuxième ensemble de pistes conductrices étant en communication électrique avec une ligne respective parmi les lignes d'électrodes de ligne .

Selon un mode de réalisation, chaque contact d'électrode de colonne de la première ligne externe de contacts d'électrode de colonne est connecté électriquement à l'électrode de colonne respective correspondante de l'ensemble d'électrodes de colonne par un connecteur d'électrode de colonne respectif d'un premier ensemble de connecteurs d'électrode de colonne formé dans une première couche métallique de surface latérale recouvrant une première surface latérale de la plaque de matériau actif, les connecteurs d'électrode de colonne du premier ensemble de connecteurs d'électrode de colonne étant individualisés par les encoches de découpe du premier ensemble d'encoches de découpe.

Selon un mode de réalisation, chaque contact d'électrode de colonne de la première ligne externe de contacts d'électrode de colonne est connecté électriquement à l'électrode de colonne respective correspondante de l'ensemble d'électrodes de colonne par un via métallisé traversant verticalement la plaque de matériau actif.

Selon un mode de réalisation, le substrat de la couche d'interconnexion comporte une surface interne, le premier ensemble de pistes conductrices et le deuxième ensemble de pistes conductrices sont sur la surface interne du substrat, et le premier ensemble de pistes conductrices forme une première distribution d'interconnexions de la couche d'interconnexion, et le deuxième ensemble de pistes conductrices forme une deuxième distribution d'interconnexions de la couche d'interconnexion.

Selon un mode de réalisation, le substrat de la couche d'interconnexion comporte une surface interne, une surface externe, des vias alignés respectivement avec la première ligne externe de contacts d'électrode de colonne, et des connecteurs de vias respectifs dans les vias, le premier ensemble de pistes conductrices est sur la surface externe du substrat et est en communication électrique avec des contacts d'électrode de colonne respectifs de la première ligne externe de contacts d'électrode de colonne au moyen de connecteurs de vias, et le deuxième ensemble de pistes conductrices est sur la surface interne du substrat, et le premier ensemble de pistes conductrices et le deuxième ensemble de pistes conductrices forment une première distribution d'interconnexions de la couche d'interconnexion.

Selon un mode de réalisation, le deuxième ensemble d'encoches de découpe parallèles individualise en outre une deuxième ligne externe de contacts d'électrode de colonne, chaque contact d'électrode de colonne de la première ligne externe de contacts d'électrode de colonne étant connecté électriquement à une électrode de colonne respective de l'ensemble d'électrodes de colonne.

Selon un mode de réalisation, les première et deuxième directions sont orthogonales.

Selon un mode de réalisation, le premier ensemble d'encoches de découpe et le deuxième ensemble d'encoches de découpe sont remplis avec un matériau polymère.

Selon un mode de réalisation, la couche d'interconnexion est une carte de circuit imprimé flexible.

Selon un mode de réalisation, le dispositif comprend en outre un ensemble de couches de face arrière fixées à la couche d'interconnexion.

Un autre mode de réalisation prévoit un procédé de fabrication d'un dispositif de transduction ultrasonore matriciel à adressage ligne-colonne, le procédé comprenant :
la fourniture d'une plaque brute de matériau actif métallisée comportant une plaque de matériau actif ayant une première surface principale et une deuxième surface principale à l'opposé de la première surface principale, une première couche métallique de surface principale sur la première surface principale, et une deuxième couche métallique de surface principale sur la deuxième surface principale, la plaque brute de matériau actif métallisée ayant une largeur Wp et une longueur Lp ;
la découpe de la plaque brute de matériau actif métallisée pour individualiser une plaque de matériau actif métallisée à partir de la plaque brute de matériau actif métallisée ;
la fixation d'une couche d'adaptation d'impédance acoustique à la deuxième couche métallique de surface principale de la plaque de matériau actif métallisée ;
la découpe complète de la plaque de matériau actif métallisée dans une première direction pour former un premier ensemble d'encoches de découpe parallèles, le premier ensemble d'encoches de découpe formant dans la deuxième couche métallique de surface principale un ensemble d'électrodes de colonne ;
la découpe partielle de la plaque de matériau actif métallisée dans une deuxième direction pour former un deuxième ensemble d'encoches de découpe parallèles, le deuxième ensemble d'encoches de découpe formant dans la première couche métallique de surface principale des lignes d'électrodes de ligne et une première ligne externe de contacts d'électrode de colonne, chaque contact d'électrode de colonne de la première ligne externe de contacts d'électrode de colonne étant connecté électriquement à une électrode de colonne respective de l'ensemble d'électrodes de colonne ;
la fourniture d'une couche d'interconnexion comportant un substrat d'un matériau électriquement isolant, un premier ensemble de pistes conductrices sur le substrat électriquement isolant, un deuxième ensemble de pistes conductrices sur le substrat électriquement isolant ;
la connexion de chaque piste du premier ensemble de pistes conductrices pour qu'elle soit en communication électrique avec un contact d'électrode de colonne respectif de la première ligne externe de contacts d'électrode de colonne ; et
la connexion de chaque piste du deuxième ensemble de pistes conductrices en contact électrique avec une ligne respective parmi les lignes d'électrodes de ligne.

Selon un mode de réalisation, chaque contact d'électrode de colonne de la première ligne externe de contacts d'électrode de colonne est connecté électriquement à l'électrode de colonne respective correspondante de l'ensemble d'électrodes de colonne par un connecteur d'électrode de colonne respectif d'un premier ensemble de connecteurs d'électrode de colonne formé dans une première couche métallique de surface latérale de la plaque de matériau actif métallisée, recouvrant une première surface latérale de la plaque de matériau actif, les connecteurs d'électrode de colonne du premier ensemble de connecteurs d'électrode de colonne étant individualisés par les encoches de découpe du premier ensemble d'encoches de découpe.

Selon un mode de réalisation, la plaque de matériau actif métallisée a une longueur La inférieure à la longueur Lp de la plaque brute de matériau actif métallisée et une largeur Wa égale à la largeur Wp de la plaque brute de matériau actif métallisée.

Selon un mode de réalisation, chaque contact d'électrode de colonne de la première ligne externe de contacts d'électrode de colonne est connecté électriquement à l'électrode de colonne respective correspondante de l'ensemble d'électrodes de colonne par un via métallisé de la plaque de matériau actif métallisée, traversant verticalement la plaque de matériau actif.

Selon un mode de réalisation, la plaque de matériau actif métallisée a une longueur La inférieure à la longueur Lp de la plaque brute de matériau actif métallisée et une largeur Wa inférieure à la largeur Wp de la plaque brute de matériau actif métallisée.

### Brève description des dessins

Des modes de réalisation seront décrits ci-après en relation avec les figures jointes et des exemples fournis à titre illustratif sans pour autant limiter la portée des revendications :
La figure 1 est un schéma simplifié d'une structure éclatée d'interconnexion classique d'un dispositif de transduction ultrasonore RCA.
La figure 2 est une vue en perspective d'une plaque brute de matériau actif selon l'invention.
La figure 3 est une vue en perspective de la plaque brute de matériau actif de la figure 2 après métallisation pour former une plaque de matériau actif métallisée.
La figure 4 est une vue en perspective d'une plaque de matériau actif métallisée résultant de la découpe de la plaque brute de matériau actif métallisée de la figure 3.
La figure 5 est une vue en perspective de la plaque de matériau actif métallisée de la figure 4 fixée à une couche d'adaptation d'impédance acoustique pour former un ensemble intermédiaire.
La figure 6 est une vue en perspective de l'ensemble intermédiaire de la figure 5 après la découpe complète de la plaque de matériau actif métallisée dans une première direction pour former un premier ensemble de traits de scie parallèles.
La figure 7 est une vue en perspective de l'ensemble intermédiaire de la figure 6 après la découpe partielle de la plaque de matériau actif métallisée dans une deuxième direction pour former un deuxième ensemble de traits de scie parallèles.
La figure 8 est une vue en perspective de l'ensemble intermédiaire de la figure 7 après fixation d'une couche d'interconnexion.
La figure 9 est une vue agrandie d'une partie correspondante de l'ensemble intermédiaire de la figure 8.
La figure 10 est une vue en perspective d'un transducteur en réseau de lignes et de colonnes assemblé selon l'invention.
La figure 11 est une vue en perspective de l'ensemble intermédiaire après la découpe partielle d'une plaque de matériau actif métallisée pour créer des traits de scie, dans lequel les traits de scie sont remplis avec un matériau polymère selon une variante de mode de réalisation de l'invention.
La figure 12 est une vue en perspective de l'ensemble intermédiaire de la figure 11 après fixation d'une couche d'interconnexion.
La figure 13 est une vue en perspective d'un ensemble intermédiaire selon un autre mode de réalisation de l'invention, dans lequel un premier ensemble de pistes conductrices se trouve sur une surface externe d'un substrat d'une couche d'interconnexion et un deuxième ensemble de pistes conductrices se trouve sur une surface interne du substrat de la couche d'interconnexion.
La figure 14 est une vue en perspective d'une plaque de matériau actif après métallisation selon un autre mode de réalisation.
La figure 15 est une vue en perspective d'une plaque de matériau actif métallisée résultant de la découpe de la plaque de matériau actif métallisée de la figure 14.
La figure 16 est une vue en perspective d'un ensemble intermédiaire après la découpe partielle de la plaque de matériau actif métallisée de la figure 15 dans des première et deuxième directions.

### Description des modes de réalisation

Les détails d'un ou de plusieurs modes de réalisation de l'objet décrit ici sont exposés dans ce document. Des modifications aux modes de réalisation décrits dans ce document, ainsi que d'autres modes de réalisation, seront évidents à la personne du métier après l'étude des informations données dans ce document. Les informations données dans ce document et, en particulier, les détails spécifiques des exemples de mode de réalisation décrits sont fournis essentiellement par souci de clarté et pour faciliter la compréhension et sont non limitatifs. En cas de conflit, la description de ce document, y compris les définitions, sera prioritaire.

Bien que les termes suivants soient supposés bien connus de la personne du métier, des définitions sont données pour faciliter l'explication de l'objet décrit ici.

Sauf précision contraire, tous les termes techniques et scientifiques utilisés ici ont le même sens que celui communément compris de la personne du métier compétente dans le domaine concerné. De même, les procédés, dispositifs et matériaux décrits ici permettent la mise en œuvre ou le test de la présente invention, bien que d'autres procédés, dispositifs, et matériaux similaires ou équivalents à ceux décrits ici puissent être utilisés pour aboutir aux mêmes fins.

Les termes "un", "une", et "le" désignent "un ou plusieurs" lorsqu'ils sont utilisés dans cette demande, y compris dans les revendications. Ainsi, par exemple, la référence à "un transducteur" inclut une pluralité de tels transducteurs et ainsi de suite.

Sauf précision contraire, tous les nombres exprimant des composantes de composition, des propriétés telles que la fréquence, etc., utilisés dans la description et les revendications doivent être compris comme étant modifiés en toutes circonstances par le terme "environ". Par conséquent, sauf précision contraire, les paramètres numériques indiqués dans ces description et revendications sont des approximations qui peuvent varier en fonction des propriétés souhaitées que l'on cherche à obtenir par l'objet décrit ici.

Tel qu'employé ici, le terme "environ", lorsqu'il est affecté à une valeur ou à une quantité est supposé incorporer des variations de ±20 % dans certains modes de réalisation, de ±10 % dans certains modes de réalisation, de ±5 % dans certains modes de réalisation, de ±1 % dans certains modes de réalisation, de ±0,5 % dans certains modes de réalisation, et de ±0,1 % dans certains modes de réalisation par rapport à la quantité indiquée, car de telles variations sont appropriées pour mettre en œuvre l'objet décrit.

Tel qu'utilisé ici, des plages peuvent être exprimées comme partant "d'environ" une valeur particulière, et/ou jusqu'à "environ" une autre valeur particulière. On comprendra également qu'il y a un certain nombre de valeurs décrites ici, et que chaque valeur est également décrite ici comme étant "environ" cette valeur particulière en plus de la valeur elle-même. Par exemple, si la valeur "10" est décrite, alors "environ 10" est également décrite. On comprendra également que chaque unité comprise entre deux unités particulières est également décrite. Par exemple, si 10 et 15 sont décrits, alors 11, 12, 13 et 14 sont également décrites.

Les termes "matrice de transducteurs " ou "réseau de transducteurs" ou "dispositif de transduction ultrasonore" sont utilisés ici pour décrire un dispositif transducteur obtenu par un agencement géométrique d'une pluralité de transducteurs (c'est-à-dire d'éléments transducteurs) ayant des dimensions compatibles avec les caractéristiques de focalisation et de déflexion d'un faisceau ultrasonore.

Les termes "transducteur élémentaire" ou "élément transducteur" ou "transducteur" sont utilisés ici pour décrire un composant transducteur ultrasonore individuel d'une matrice de transducteurs. Généralement, un transducteur élémentaire d'une matrice de transducteurs a des dimensions planes appropriées pour une focalisation et une déflexion électroniques de faisceaux ultrasonores. Chaque élément transducteur ou transducteur élémentaire est muni de deux électrodes conductrices. Une électrode conductrice peut être soit formée par des procédés tant soustractifs qu'additifs dans le cas d'un réseau d'éléments sans découpe, soit gravée en même temps que la couche de matériau actif pendant le procédé d'individualisation des éléments.

Un dispositif de transduction ultrasonore à adressage ligne-colonne (RCA), comme cela a été présenté précédemment, est un transducteur en matrice ayant "R" lignes d'électrodes et "C" colonnes d'électrodes sur des côtés opposés d'un matériau actif, où un réseau d'interconnexion de ligne R comporte des pistes de ligne qui sont connectées aux lignes sur une première surface principale de la matrice de transducteurs et un réseau d'interconnexion de colonne C est connecté aux colonnes sur une seconde surface principale de la matrice opposée à la première surface. Des éléments transducteurs individuels sont actionnés en appliquant des signaux appropriés sur une piste de ligne du réseau d'interconnexion de ligne R et sur une piste de colonne du réseau d'interconnexion de colonne C correspondant à l'élément transducteur en cours d'activation.

Tel qu'utilisé ici, le terme "matériau actif" signifie un matériau de transducteur ultrasonore qui subit des compressions et des étirements afin de convertir une énergie électrique en ultrasons ou inversement. Des exemples de tels matériaux actifs incluent un matériau piézoélectrique tel que du PZT, du KNN, du BaTiO3, du PMN-PT, du LNO3 soit sous forme de matériau céramique ou monocristallin, soit constituant l'ensemble du volume du matériau actif soit sous forme composite (mélangé à un matériau polymère), aussi bien qu'un polymère piézoélectrique tel que le PVDF. Une "plaque de matériau actif" est une feuille mince et plate d'un matériau actif. Une "plaque brute de matériau actif" est une grande feuille mince et plate d'un matériau actif à partir de laquelle des plaques plus petites du matériau actif sont découpées ou individualisées d'une autre façon.

Tel qu'utilisé ici, le terme "couche d'adaptation d'impédance acoustique" ou bien "couche d'adaptation" signifie une couche ou des couches d'un matériau fixé d'un côté avant du matériau actif du transducteur ultrasonore ayant une impédance acoustique dont la valeur est entre celle de l'impédance acoustique du matériau actif et celle de l'impédance acoustique de l'élément à imager (par exemple un tissu humain). Cette couche d'adaptation d'impédance permet de minimiser la réflexion de l'onde ultrasonore au niveau de l'interface entre le matériau actif et le milieu à imager, par exemple le corps d'un patient.

Tel qu'utilisé ici, le terme "matériau arrière" ou "couche de face arrière" ou, selon la terminologie courante en langue anglaise "backing" signifie une couche ou des couches d'un ou plusieurs matériaux d'amortissement acoustique fixés à une face arrière du matériau actif du transducteur ultrasonore pour éviter des réflexions parasites d'ondes acoustiques.

Tel qu'utilisé ici, les termes "couper" et "découper" signifient couper un matériau ou plusieurs matériaux assemblés entre eux pour former des tranchées ou traits de scie dans le matériau actif ou pour individualiser une plaque de matériau actif à partir d'une plaque brute. Un moyen préféré pour couper ou découper est une scie à fil diamanté circulaire à rotation. "Couper" et "découper" peuvent également inclure une découpe au laser ou une gravure chimique en tant que moyens pour couper ou découper.

Tel qu'utilisé ici, le terme "trait de scie" ("kerf" en langue anglaise) signifie une fente ou une encoche formée en coupant, par exemple avec une scie ou d'autres moyens de coupe (voir "découper").

Tel qu'utilisé ici, le terme "direction C" signifie une direction de colonne de la matrice de transducteur élémentaires.

Tel qu'utilisé ici, le terme "direction R" signifie une direction de ligne de la matrice de transducteurs élémentaires.

Tel qu'utilisé ici, le terme "couche d'interconnexion" signifie une couche de matériau isolant sur laquelle des pistes électriques sont formées pour interconnecter des électrodes d'éléments individuels d'un dispositif de transduction RCA avec un système d'imagerie.

Tel qu'utilisé ici, le terme "distribution" ("fan-out" en langue anglaise) ou "distribution d'interconnexions" ("interconnect fan-out" en anglais) désigne les interconnexions électriques entre les éléments transducteurs et les connexions électriques amont afin d'adapter le pas et la direction des contacts électriques au niveau de chaque extrémité de l'interconnexion. La distribution d'interconnexions est obtenue par exemple avec une carte de circuit imprimé flexible.

Tel qu'utilisé ici, le terme "film conducteur anisotrope" signifie un système d'interconnexion adhésif pour former des connexions électriques et mécaniques allant depuis des circuits électroniques de commande jusqu'à des substrats.

Le terme "système d'imagerie" est utilisé ici pour décrire un appareil qui comporte un générateur de signal, un processeur de signal et une interface utilisateur. Le générateur de signal est destiné à générer des signaux d'activation d'éléments transducteurs d'un transducteur ultrasonore pour générer des ondes acoustiques. Le processeur de signal est destiné à traiter les signaux générés par les éléments transducteurs en réponse à des ondes acoustiques reçues par les éléments transducteurs. Le processeur de signal comporte en outre un processeur d'images destiné à générer des images à partir des signaux traités. L'interface utilisateur est destinée à afficher les images générées et à recevoir des entrées supplémentaires en provenance d'un utilisateur du système d'imagerie. De tels systèmes d'imagerie sont commercialement disponibles et connus de la personne du métier, de sorte que les détails des éléments et le fonctionnement de tels systèmes d'imagerie ne seront pas décrits plus ici.

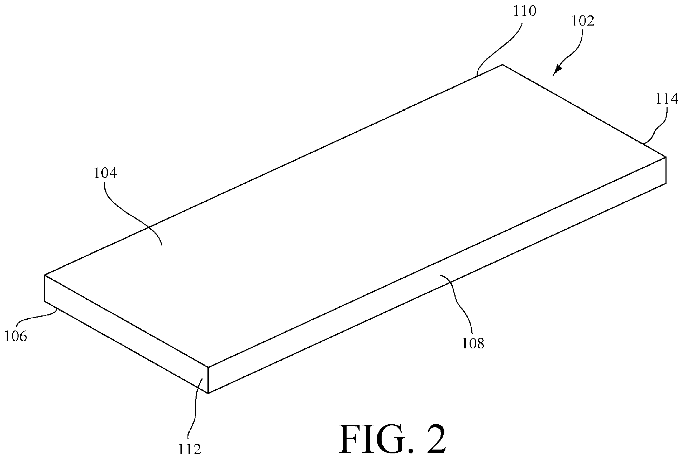

Un premier exemple de procédé de fabrication d'un transducteur ultrasonore en réseau de lignes et de colonnes selon l'invention commence par la fourniture, comme cela est illustré en figure 2, d'une plaque brute de matériau actif 102 ayant une première surface principale 104 et une deuxième surface principale 106 à l'opposé à la première surface principale 104. Sur des côtés s'étendant entre la première surface principale 104 et la deuxième surface principale 106, la plaque brute de matériau actif 102 a également une première surface latérale 108, une deuxième surface latérale 110 opposée ou en vis-à-vis de la première surface latérale 108, une troisième surface latérale 112, et une quatrième surface latérale 114 opposée ou en vis-à-vis de la troisième surface latérale 112.

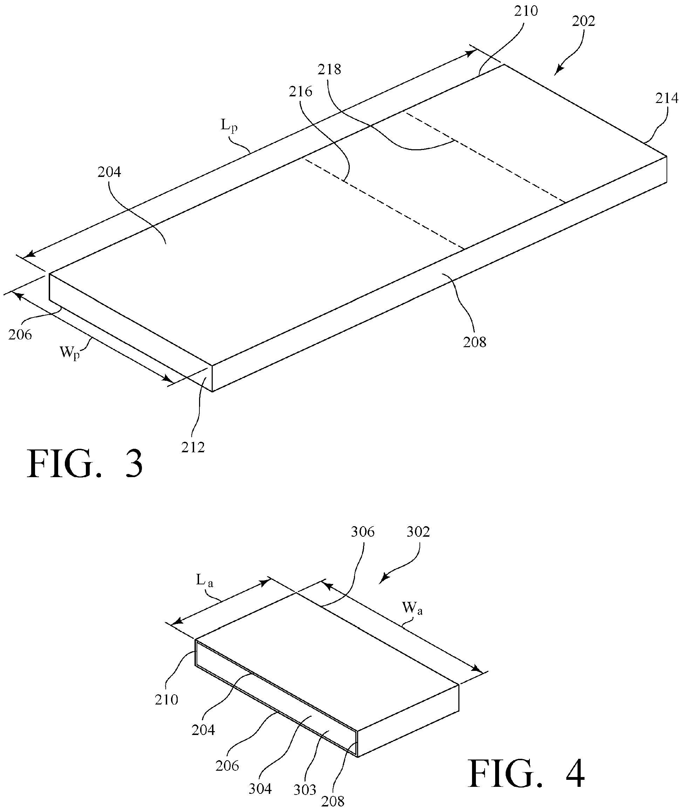

La plaque brute de matériau actif 102 est ensuite métallisée pour former une plaque brute de matériau actif métallisée 202, comme cela est représenté en figure 3. Apparaissent en figure 3 une première couche métallique de surface principale 204, une deuxième couche métallique de surface principale 206, une première couche métallique de surface latérale 208, une deuxième couche métallique de surface latérale 210, une troisième couche métallique de surface latérale 212, et une quatrième couche métallique de surface latérale 214. Comparativement à la plaque brute de matériau actif 102 (figure 2), la première couche métallique de surface principale 204 est sur la première surface principale 104, la deuxième couche métallique de surface principale 206 est sur la deuxième surface principale 106, la première couche métallique de surface latérale 208 est sur la première surface latérale 108, la deuxième couche métallique de surface latérale 210 est sur la deuxième surface latérale 110, la troisième couche métallique de surface latérale 212 est sur la troisième surface latérale 112, et la quatrième couche métallique de surface latérale 214 est sur la quatrième surface latérale 114. La première couche métallique de surface latérale 208, la deuxième couche métallique de surface latérale 210, la troisième couche métallique de surface latérale 212, et la quatrième couche métallique de surface latérale 214 sont connectées électriquement à la première couche métallique de surface principale 204 et à la deuxième couche métallique de surface principale 206. Cela est désigné ci-après comme étant une métallisation "enveloppante". La plaque brute de matériau actif 102 (figure 2) est par conséquent entièrement métallisée, bien qu'une seule parmi la première couche métallique de surface latérale 208 et la deuxième couche métallique de surface latérale 210 soit nécessaire pour assurer une seule couche d'interconnexion pour à la fois les électrodes de réseau R et les électrodes de réseau C, comme cela sera discuté ultérieurement. La plaque brute de matériau actif métallisée 202 a une largeur, Wp, et une longueur, Lp.

Un exemple de procédé de métallisation d'une plaque de matériau actif comporte le dépôt d'une ou de plusieurs couches de matériaux électriquement conducteurs, par exemple de l'or (Au) ou du cuivre (Cu), du nickel (Ni) ou de l'argent (Ag). Le procédé de dépôt est obtenu par exemple par dépôt physique en phase vapeur (PVD) ou par dépôt chimique en phase vapeur (CVD). Ainsi, la première couche métallique de surface principale 204, la deuxième couche métallique de surface principale 206, et les couches métalliques de surface latérale 208, 210, 212, 214 sont des couches très minces allant de quelques dizaines (par exemple, 20 à 40) de nanomètres jusqu'à quelques (par exemple, 2 à 4) micromètres comparé aux dimensions de la plaque brute de matériau actif dont l'épaisseur est dans la plage allant de quelques dizaines (par exemple, 20 à 40) de micromètres jusqu'à quelques (par exemple, 2 à 4) millimètres et des dimensions latérales allant de quelques (par exemple, 2 à 4) millimètres à des centaines de millimètres. Ainsi, même si la plaque brute de matériau actif 102 est sous la première couche métallique de surface principale 204, la deuxième couche métallique de surface principale 206, et les couches métalliques de surface latérale 208, 210, 212, 214 et n'est par conséquent pas visible en figure 3, la personne du métier comprendra que les largeur, longueur, et épaisseur de la plaque brute de matériau actif 102 sont sensiblement les mêmes que les largeur, Wp, longueur, Lp, et épaisseur de la plaque brute de matériau actif métallisée 202.

Le premier exemple de procédé de fabrication d'un transducteur ultrasonore selon l'invention comporte ensuite une étape de découpe de la plaque brute de matériau actif métallisée 202 dans la direction de la largeur au niveau de deux emplacements de coupe 216, 218 pour individualiser une plaque de matériau actif métallisée 302, comme cela est représenté en figure 4, à partir de la plaque brute de matériau actif métallisée 202. La plaque de matériau actif métallisée 302 a une largeur Wa égale à Wp, une longueur La inférieure à la longueur Lp, et une épaisseur égale à l'épaisseur de la plaque brute de matériau actif métallisée 202. La longueur La est égale à la longueur finale du dispositif de transduction ultrasonore RCA.

Au centre de la plaque de matériau actif métallisée 302 se trouve une plaque de matériau actif 303 individualisée à partir de la plaque brute de matériau actif 102 (figure 2). La plaque de matériau actif 303 a une première surface principale correspondant à la première surface principale 104 de la plaque brute de matériau actif 102, une deuxième surface principale à l'opposé de la première surface principale et correspondant à la deuxième surface principale 106 de la plaque brute de matériau actif 102, une première surface latérale s'étendant entre la première surface principale et la deuxième surface principale et correspondant à la première surface latérale 108 de la plaque brute de matériau actif 102, et une deuxième surface latérale correspondant à la deuxième surface latérale 110 de la plaque brute de matériau actif 102. L'épaisseur de la plaque de matériau actif 303 (c'est-à-dire l'épaisseur de la plaque de matériau actif) correspond à l'épaisseur de plaque brute de matériau actif. La première couche métallique de surface principale 204, la deuxième couche métallique de surface principale 206, la première couche métallique de surface latérale 208 et la deuxième couche métallique de surface latérale 210 recouvrent la première surface principale, la deuxième surface principale, la première surface latérale et la deuxième surface latérale, respectivement, de la plaque de matériau actif 303. Par conséquent, la première surface principale, la deuxième surface principale, la première surface latérale et la deuxième surface latérale de la plaque de matériau actif 303 ne sont pas visibles en figure 4.

La figure 4 illustre la plaque de matériau actif 303 ayant des surfaces latérales exposées 304, 306. La figure 4 illustre également des surfaces latérales exposées de la première couche métallique de surface principale 204, la deuxième couche métallique de surface principale 206, la première couche métallique de surface latérale 208 et la deuxième couche métallique de surface latérale 210. Avantageusement, la première couche métallique de surface latérale 208 et la deuxième couche métallique de surface latérale 210 connectent toujours électriquement la première couche métallique de surface principale 204 et la deuxième couche métallique de surface principale 206 (c'est-à-dire une métallisation "enveloppante" de la plaque de matériau actif 303 également désignée par le terme anglais de "wrap-around").

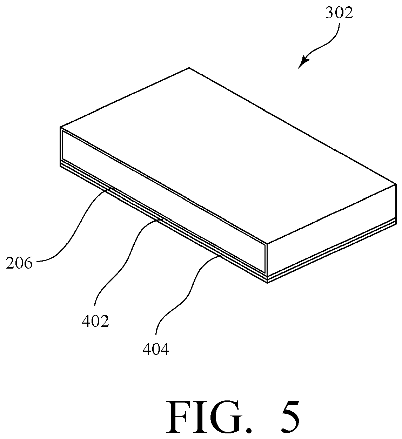

La figure 5 illustre la plaque de matériau actif métallisée 302 après la fixation d'une couche d'adaptation d'impédance acoustique 402 à sa deuxième couche métallique de surface principale 206 pour former un ensemble intermédiaire. En option, si cela est prévu dans la structure d'empilement acoustique, une couche (ou des couches) d'adaptation d'impédance acoustique supplémentaire(s) 404 pourrai(en)t également être fixée(s) à la couche d'adaptation d'impédance acoustique 402. Bien que cela ne soit pas représenté en figure 5, l'empilement acoustique peut également comporter une couche de désadaptation d'impédance qui forme un miroir acoustique ("dematching layer" en anglais) - fixée à la première couche métallique de surface principale 204 de la plaque de matériau actif 302.

La couche d'adaptation d'impédance acoustique 402 et la couche (ou les couches) d'adaptation d'impédance acoustique 404 sont de préférence constituées d'un matériau polymère chargé de particules afin d'obtenir les propriétés acoustiques souhaitées (par exemple, de vitesse du son), ou des couches de graphite.

La fixation de la couche d'adaptation d'impédance acoustique 402 à la deuxième couche métallique de surface principale 206 est de préférence effectuée par collage adhésif en déposant une couche mince de colle entre celles-ci et en la polymérisant par traitement thermique (allant de la température ambiante à 100 °C) et sous une pression appliquée à l'empilement. Selon une variante, la fixation est obtenue par un processus de collage moléculaire ("wafer bonding").

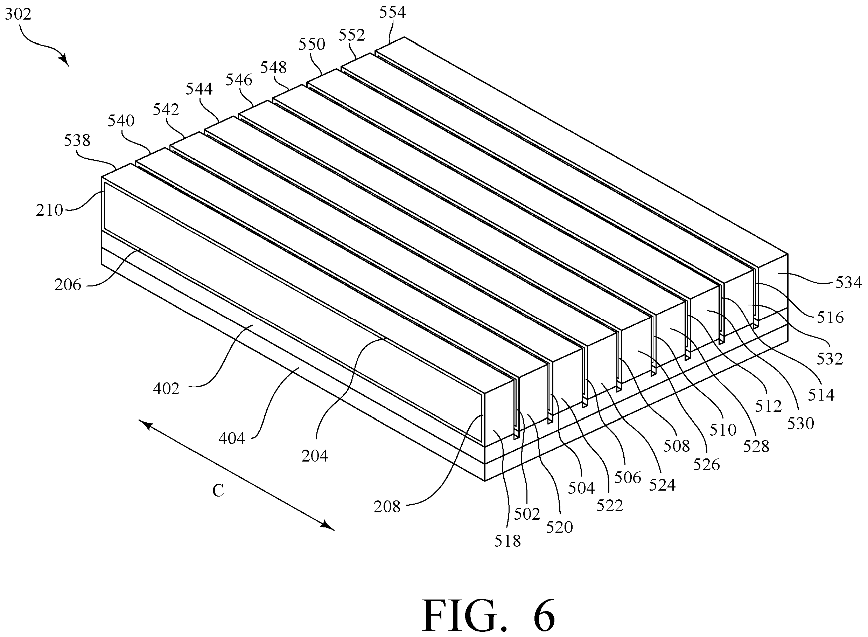

La figure 6 illustre l'ensemble intermédiaire après la découpe complète de la plaque de matériau actif métallisée 302 (c'est-à-dire la découpe complète à travers une épaisseur de la plaque de matériau actif métallisée 302) dans une première direction (c'est-à-dire une direction C comme cela est indiqué par les flèches directionnelles référencées "C") pour former un premier ensemble de traits de scie 502, 504, 506, 508, 510, 512, 514, 516. Le premier ensemble de traits de scie 502, 504, 506, 508, 510, 512, 514, 516 forme dans la deuxième couche métallique de surface principale 206 un ensemble d'électrodes de colonne au niveau de l'interface de la deuxième couche métallique de surface principale 206 et de la couche d'adaptation d'impédance acoustique 402, et forme dans la première couche métallique de surface latérale 208 un premier ensemble de connecteurs d'électrode de colonne 518, 520, 522, 524, 526, 528, 530, 532, 534 qui connectent électriquement les électrodes de colonne à la première couche métallique de surface principale 204. De façon correspondante, le premier ensemble de traits de scie 502, 504, 506, 508, 510, 512, 514, 516 forme également dans la deuxième couche métallique de surface latérale 210 un deuxième ensemble de connecteurs d'électrode de colonne 538, 540, 542, 544, 546, 548, 550, 552, 554. Le premier ensemble de connecteurs d'électrode de colonne 518, 520, 522, 524, 526, 528, 530, 532, 534 et le deuxième ensemble de connecteurs d'électrode de colonne 538, 540, 542, 544, 546, 548, 550, 552, 554 connectent électriquement les électrodes de colonne à la première couche métallique de surface principale 204. La couche d'adaptation d'impédance acoustique 402 sert à maintenir en place la plaque de matériau actif métallisée totalement découpée 302.

Selon une variante, la profondeur de découpe du premier ensemble de traits de scie 502, 504, 506, 508, 510, 512, 514, 516 peut également s'étendre à travers les couches d'adaptation d'impédance 402 et 404 (seulement une découpe partielle de la couche d'adaptation d'impédance 402 est représenté en figure 6). Cette extension de profondeur est nécessaire si la couche d'adaptation d'impédance 402 ou les couches d'adaptation d'impédance 402 et 404 sont conductrices électriquement afin d'éviter un court-circuit entre les électrodes de colonne. Dans ce cas, l'ensemble de l'empilement est préalablement fixé sur un substrat de maintien mécanique provisoire qui est ensuite retiré après la dernière étape de fabrication du transducteur.

La première direction (c'est-à-dire la direction C) est représentée en figure 6 comme étant la direction de largeur, sans que cela soit limitatif. La seule limite importante sur la première direction est qu'elle amène le premier ensemble de traits de scie 502, 504, 506, 508, 510, 512, 514, 516 à former dans la deuxième couche métallique de surface principale 206 le premier ensemble d'électrodes de colonne au niveau de l'interface de la deuxième couche métallique de surface principale 206 et de la couche d'adaptation d'impédance acoustique 402, et à former dans la première couche métallique de surface latérale 208 le premier ensemble de connecteurs d'électrode de colonne 518, 520, 522, 524, 526, 528, 530, 532, 534.

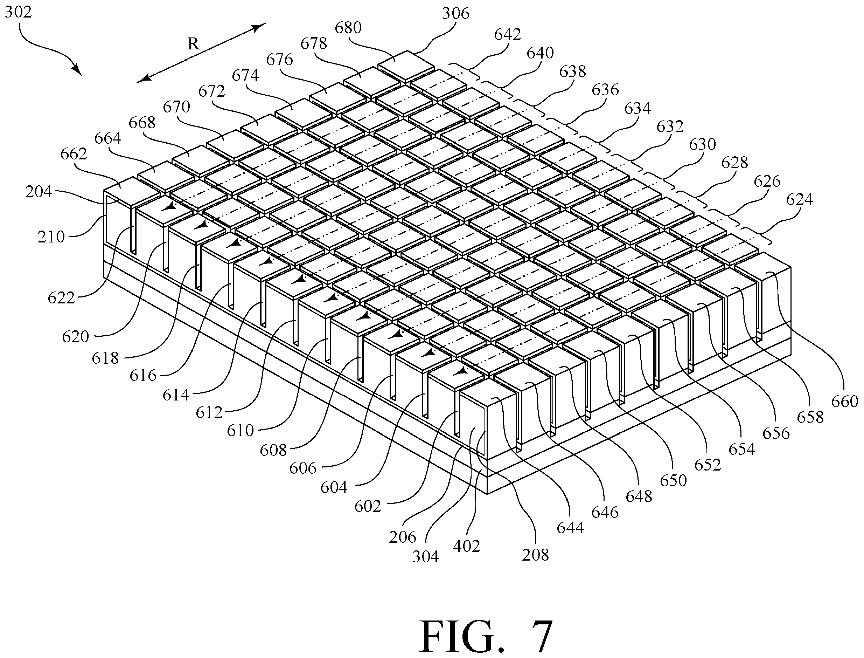

La figure 7 illustre l'ensemble intermédiaire après une coupe partielle de la plaque de matériau actif métallisée 302 (c'est-à-dire en découpant moins que l'épaisseur de la plaque de matériau actif 303) dans une deuxième direction (c'est-à-dire une direction R, comme cela est indiqué par les flèches directionnelles référencées "R") pour former un deuxième ensemble de traits de scie 602, 604, 606, 608, 610, 612, 614, 61, 618, 620, 622. La profondeur de découpe doit être inférieure à l'épaisseur de la plaque de matériau actif 303 pour préserver la continuité électrique de l'ensemble d'électrodes de colonne au niveau de l'interface de la deuxième couche métallique de surface principale 206 et de la couche d'adaptation d'impédance acoustique 402. Le deuxième ensemble de traits de scie 602, 604, 606, 608, 610, 612, 614, 616, 618, 620, 622 forme dans la première couche métallique de surface principale 204 des lignes d'électrodes de ligne 624, 626, 628, 630, 632, 634, 636, 638, 640, 642, une première ligne externe de contacts d'électrode de colonne 644, 646, 648, 650, 652, 654, 656, 658, 660, et une deuxième ligne externe de contacts d'électrode de colonne 662, 664, 668, 670, 672, 674, 676, 678, 680. La première ligne externe de contacts d'électrode de colonne 644, 646, 648, 650, 652, 654, 656, 658, 660 est connectée électriquement respectivement au premier ensemble de connecteurs d'électrode de colonne 518, 520, 522, 524, 526, 528, 530, 532, 534 (figure 6), et la deuxième ligne externe de contacts d'électrode de colonne 662, 664, 668, 670, 672, 674, 676, 678, 680 est connectée électriquement respectivement au deuxième ensemble de connecteurs d'électrode de colonne 538, 540, 542, 544, 546, 548, 550, 552, 554 (figure 6). Ainsi, la métallisation "enveloppante" décrite précédemment de la plaque de matériau actif 303 (figure 4) permet à l'ensemble d'électrodes de colonne (c'est-à-dire les électrodes de réseau C) au niveau de l'interface de la deuxième couche métallique de surface principale 206 et de la couche d'adaptation d'impédance acoustique 402 d'être connecté électriquement respectivement à la première ligne externe de contacts d'électrode de colonne 644, 646, 648, 650, 652, 654, 656, 658, 660 et à la deuxième ligne externe de contacts d'électrode de colonne 662, 664, 668, 670, 672, 674, 676, 678, 680. Avantageusement, la première ligne externe de contacts d'électrode de colonne 644, 646, 648, 650, 652, 654, 656, 658, 660 et la deuxième ligne externe de contacts d'électrode de colonne 662, 664, 668, 670, 672, 674, 676, 678, 680 forment des zones d'interconnexion particulières pour des électrodes C sur des bords opposés de la première couche métallique de surface principale 204 et du même côté de la plaque de matériau actif métallisée 302 que les lignes d'électrodes de ligne 624, 626, 628, 630, 632, 634, 636, 638, 640, 642 (c'est-à-dire les électrodes de réseau R), formant une seule couche d'interconnexion pour à la fois les électrodes de réseau R et les électrodes de réseau C.

On notera qu'une seule parmi la première ligne externe de contacts d'électrode de colonne 644, 646, 648, 650, 652, 654, 656, 658, 660 et la deuxième ligne externe de contacts d'électrode de colonne 662, 664, 668, 670, 672, 674, 676, 678, 680, et seulement un connecteur correspondant parmi le premier ensemble de connecteurs d'électrode de colonne 518, 520, 522, 524, 526, 528, 530, 532, 534 et le deuxième ensemble de connecteurs d'électrode de colonne 538, 540, 542, 544, 546, 548, 550, 552, 554 (figure 6) est nécessaire pour contacter les électrodes de réseau C. Toutefois fournir à la fois la première ligne externe de contacts d'électrode de colonne 644, 646, 648, 650, 652, 654, 656, 658, 660 et la deuxième ligne externe de contacts d'électrode de colonne 662, 664, 668, 670, 672, 674, 676, 678, 680 (et les premier ensemble de connecteurs d'électrode de colonne 518, 520, 522, 524, 526, 528, 530, 532, 534 et deuxième ensemble de connecteurs d'électrode de colonne 538, 540, 542, 544, 546, 548, 550, 552, 554 correspondants (figure 6)) permet plus d'options de distribution pour la couche d'interconnexion, comme cela sera discuté ultérieurement.

On notera en outre que, dans l'ensemble intermédiaire représenté en figure 7, la découpe partielle de la plaque de matériau actif métallisée 302 pour former le deuxième ensemble de traits de scie 602, 604, 606, 608, 610, 612, 614, 61, 618, 620, 622 forme également dans la plaque de matériau actif 302 les éléments transducteurs en réseau de lignes et de colonnes (c'est-à-dire les piliers représentés en figure 7). La personne du métier comprendra que l'ordre de la coupe complète et de la coupe partielle de la plaque de matériau actif métallisée 302 pourrait être inversé, de sorte que la découpe partielle soit effectuée avant la découpe complète de la plaque de matériau actif métallisée 302. Ainsi, on comprendra que l'ordre de la coupe complète et de la coupe partielle n'a pas d'importance pour former dans la plaque de matériau actif 302 des éléments transducteurs en réseau de lignes et de colonnes (c'est-à-dire les piliers représentés en figure 7).

La deuxième direction (direction R) est orientée selon un angle d'orientation par rapport à la première direction (direction C). Comme cela est représenté, l'angle d'orientation est orthogonal ou de 90 degrés. Avantageusement, avec un angle d'orientation orthogonal, le deuxième ensemble de traits de scie 602, 604, 606, 608, 610, 612, 614, 61, 618, 620, 622 peut être limité pour s'étendre à travers les surfaces latérales exposées 34, 306 de la plaque de matériau actif 302. Toutefois, l'angle d'orientation peut comprendre d'autres angles de préférence entre, et incluant, 80 degrés et 90 degrés, de sorte que le deuxième ensemble de traits de scie 602, 604, 606, 608, 610, 612, 614, 61, 618, 620, 622 s'étende à travers les surfaces latérales exposées 304, 306 de la plaque de matériau actif 302. A titre d'exemple, un faible écart (<10°) par rapport à un angle de 90° peut être avantageux pour certaines applications d'imagerie dans lesquelles certaines directions d'imagerie sont privilégiées. L'écart par rapport à un angle de 90° ne peut pas être trop important, car le deuxième ensemble de traits de scie 602, 604, 606, 608, 610, 612, 614, 61, 618, 620, 622 ne s'étendrait plus à travers les surfaces latérales exposées 304, 306 de la plaque de matériau actif 302 (sauf à ce que les dimensions latérales du transducteur soient augmentées).

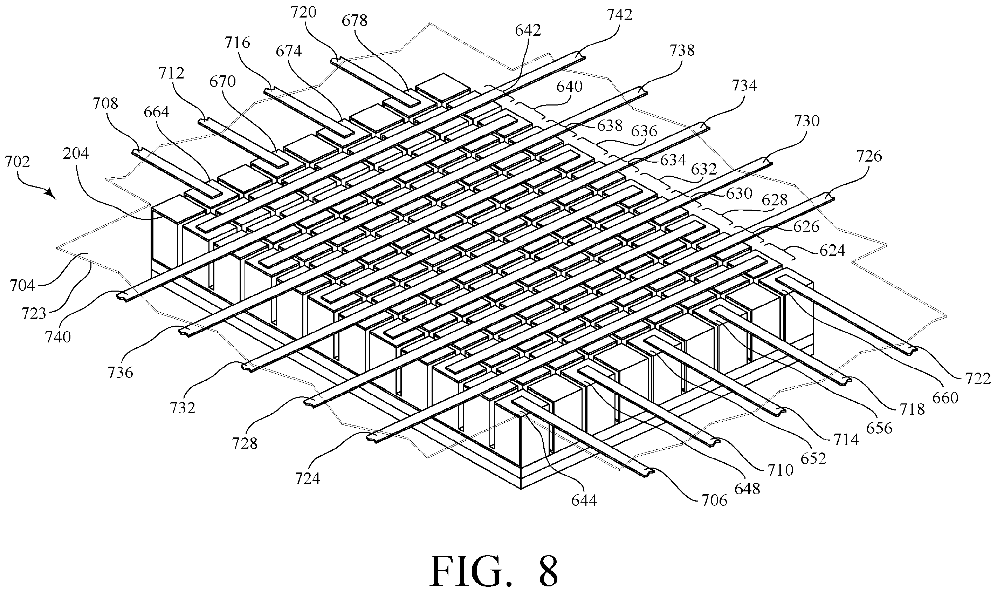

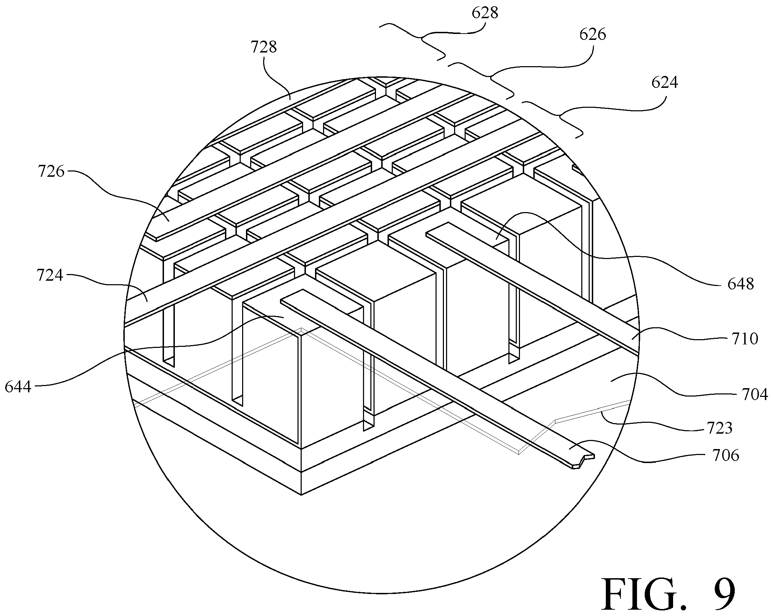

La figure 8 et la figure 9 illustrent le dispositif intermédiaire obtenu après la fixation d'une couche d'interconnexion 702 à la première couche métallique de surface principale 204. La couche d'interconnexion 702 est de préférence une carte de circuit imprimé, et peut être une carte de circuit imprimé flexible, une carte de circuit imprimé rigide, ou une carte de circuit imprimé rigide-flexible. Des cartes de circuit imprimé rigides-flexibles sont des cartes utilisant une combinaison des technologies de carte rigide et flexible dans une application.

La couche d'interconnexion 702 comporte un substrat 704 (c'est-à-dire une couche de support) qui est constitué d'un matériau isolant électriquement. Par souci de simplification et de clarté, le substrat 704 est représenté comme étant clair ou transparent, mais la personne du métier comprendra que le substrat 704 peut également être translucide ou opaque.

La couche d'interconnexion 702 comporte également un premier ensemble de pistes conductrices 706, 710, 714, 718, 722 et un troisième ensemble de pistes conductrices 708, 712, 716, 720 sur une surface interne 723 du substrat 704. La surface interne 723 du substrat 704 est dirigée vers la première couche métallique de surface principale 204 sur la plaque de matériau actif métallisée 302. Chaque piste du premier ensemble de pistes conductrices 706, 710, 714, 718, 722 est en communication électrique avec un contact d'électrode de colonne respectif de la première ligne externe de contacts d'électrode de colonne 644, 648, 652, 656, 660. Chaque piste du troisième ensemble de pistes conductrices 708, 712, 716, 720 est en communication électrique avec un contact d'électrode de colonne respectif de la deuxième ligne externe de contacts d'électrode de colonne 664, 670, 674, 678. La figure 8 illustre une connexion du premier ensemble de pistes conductrices 706, 710, 714, 718, 722 et le troisième ensemble de pistes conductrices 708, 712, 716, 720 avec des contacts respectifs à la fois de la première ligne externe de contacts d'électrode de colonne 644, 648, 652, 656, 660 et de la deuxième ligne externe de contacts d'électrode de colonne 664, 670, 674, 678 qui sont en communication électrique avec des électrodes de colonne respectives de l'ensemble des électrodes de colonne au niveau de l'interface de la deuxième couche métallique de surface principale 206 et de la couche d'adaptation d'impédance acoustique 402. Toutefois, on notera que toutes les connexions pourraient être constituées soit de la première ligne externe de contacts d'électrode de colonne 644, 646, 648, 650, 652, 654, 656, 658, 660 (voir figure 7), soit de la deuxième ligne externe de contacts d'électrode de colonne 662, 664, 668, 670, 672, 674, 676, 678, 680 (voir figure 7) et être toujours en communication électrique avec des électrodes de colonne respectives de l'ensemble d'électrodes de colonne au niveau de l'interface de la deuxième couche métallique de surface principale 206 et de la couche d'adaptation d'impédance acoustique 402.

Le premier ensemble de pistes conductrices 706, 710, 714, 718, 722 forme une première distribution d'interconnexions de la couche d'interconnexion 702, et le troisième ensemble de pistes conductrices 708, 712, 716, 720 forme une troisième distribution d'interconnexions de la couche d'interconnexion 702. Avantageusement, l'alternance des pistes à partir du premier ensemble de pistes conductrices 706, 710, 714, 718, 722 et du troisième ensemble de pistes conductrices 708, 712, 716, 720 permet un espacement plus grand entre les pistes conductrices car le pas entre les pistes conductrices est double du pas des colonnes du transducteur. Toutefois, comme cela a été décrit précédemment, les connexions électriques avec l'ensemble d'électrodes de colonne au niveau de l'interface de la deuxième couche métallique de surface principale 206 et de la couche d'adaptation d'impédance acoustique 402 pourraient être formées sur un même côté de la plaque de matériau actif métallisée 302 (c'est-à-dire une configuration non-interdigitée) en une seule distribution de la couche d'interconnexion (c'est-à-dire un "premier ensemble unique" de pistes conductrices incluant le premier ensemble de pistes conductrices 706, 710, 714, 718, 722 et le troisième ensemble de pistes conductrices 708, 712, 716, 720).

La couche d'interconnexion 702 comporte également un deuxième ensemble de pistes conductrices 724, 728, 732, 736, 740 et un quatrième ensemble de pistes conductrices 726, 730, 734, 738, 742 sur la surface interne 723 du substrat 704. Chaque piste du deuxième ensemble de pistes conductrices 724, 728, 732, 736, 740 et du quatrième ensemble de pistes conductrices 726, 730, 734, 738, 742 est en communication électrique avec une ligne respective parmi les lignes d'électrodes de ligne 624, 626, 628, 630, 632, 634, 636, 638, 640, 642.

Le deuxième ensemble de pistes conductrices 724, 728, 732, 736, 740 forme une deuxième distribution d'interconnexions de la couche d'interconnexion 702, et le quatrième ensemble de pistes conductrices 726, 730, 734, 738, 742 forme une quatrième distribution d'interconnexions de la couche d'interconnexion 702. Avantageusement, l'alternance des pistes à partir du deuxième ensemble de pistes conductrices 724, 728, 732, 736, 740 et du quatrième ensemble de pistes conductrices 726, 730, 734, 738, 742 forme une configuration interdigitée des lignes et permet un espacement plus grand entre les pistes conductrices car le pas entre les pistes conductrices est double du pas des lignes du transducteur. Toutefois, on notera que les connexions électriques avec les lignes des électrodes de ligne 624, 626, 628, 630, 632, 634, 636, 638, 640, 642 pourraient être formées sur le même côté de la plaque de matériau actif métallisée 302 (c'est-à-dire une configuration non-interdigitée) dans une seule distribution de la couche d'interconnexion (c'est-à-dire un "deuxième ensemble unique" de pistes conductrices incluant le deuxième ensemble de pistes conductrices 724, 728, 732, 736, 740 et le quatrième ensemble de pistes conductrices 726, 730, 734, 738, 742).

Par conséquent, toutes les interconnexions des éléments de ligne et de colonne du dispositif de transduction ultrasonore à adressage ligne-colonne sont réalisées sur le premier côté 723 du substrat 704 sans avoir besoin de vias ou de repliement dudit substrat. Toutefois, on notera qu'un ou les deux parmi le premier ensemble de pistes conductrices 706, 710, 714, 718, 722 et le troisième ensemble de pistes conductrices 708, 712, 716, 720 pourraient être sur une surface externe du substrat 704 et connectés à des contacts respectifs de la première ligne externe de contacts d'électrode de colonne 644, 646, 648, 650, 652, 654, 656, 658, 660 (voir figure 7), ou de la deuxième ligne externe de contacts d'électrode de colonne 662, 664, 668, 670, 672, 674, 676, 678, 680 (voir figure 7) en utilisant des vias, comme cela sera décrit ultérieurement.

Dans certains modes de réalisation, chaque piste du premier ensemble de pistes conductrices 706, 708, 710, 712, 714, 716, 718, 720, 722 et du deuxième ensemble de pistes conductrices 724, 726, 728, 730, 732, 734, 736, 738, 740, 742 sont en contact électrique avec respectivement la première ligne externe de contacts d'électrodes de colonne 644, 648, 652, 656, 660, avec la deuxième ligne externe de contacts d'électrodes de colonne 664, 670, 674, 678, et avec les électrodes de ligne 624, 626, 628, 630, 632, 634, 636, 638, 640, 642, par exemple par l'intermédiaire d'un matériau de collage, par exemple une colle ou un adhésif conducteur, ou par un film anisotrope conducteur.

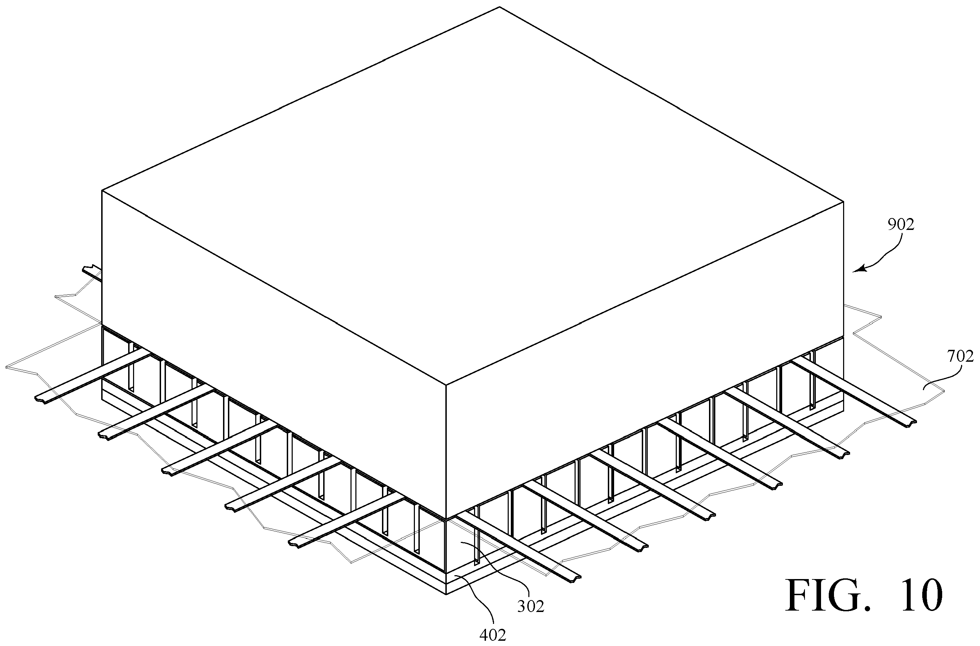

La figure 10 illustre un dispositif de transduction ultrasonore RCA assemblé comportant la plaque de matériau actif métallisée 302, la couche d'adaptation d'impédance acoustique 402, et la couche d'interconnexion 702, comme cela a été décrit précédemment, et un ensemble de couches de face arrière 902 fixé à la couche d'interconnexion 702. La fixation de l'ensemble de couches de face arrière 902 à la couche d'interconnexion 702 est effectuée de préférence par collage adhésif en déposant une colle entre eux et un procédé de polymérisation par traitement thermique (allant de la température ambiante à 100 °C) et sous une pression appliquée de l'empilement.

Dans un mode de réalisation, les couches de face arrière 902 sont des matériaux passifs ayant des propriétés acoustiques d'amortissement ou absorption tel qu'une résine époxy, soit en tant que matériau massif ou comportant des charges absorbantes ou des bulles d'air. Dans un autre mode de réalisation, les couches de face arrière comportent un circuit intégré ayant pour objet d'intégrer de manière compacte le dispositif de transduction avec son circuit de pilotage. Auquel cas, une ou plusieurs couches servant de miroir acoustique (ou "dematching layer") sont intercalées entre le circuit intégré et le reste du dispositif de transduction afin d'éviter que le circuit intégré provoque des réflexions parasites de l'onde ultrasonore vers le transducteur. Avantageusement les couches formant le miroir acoustique sont conductrices, la face principale interne vient directement en contact électrique avec les électrodes du dispositif de transduction RCA sans recours à la couche d'interconnexion 702, la face principale externe est en contact avec des plots électriques sur le circuit intégré.

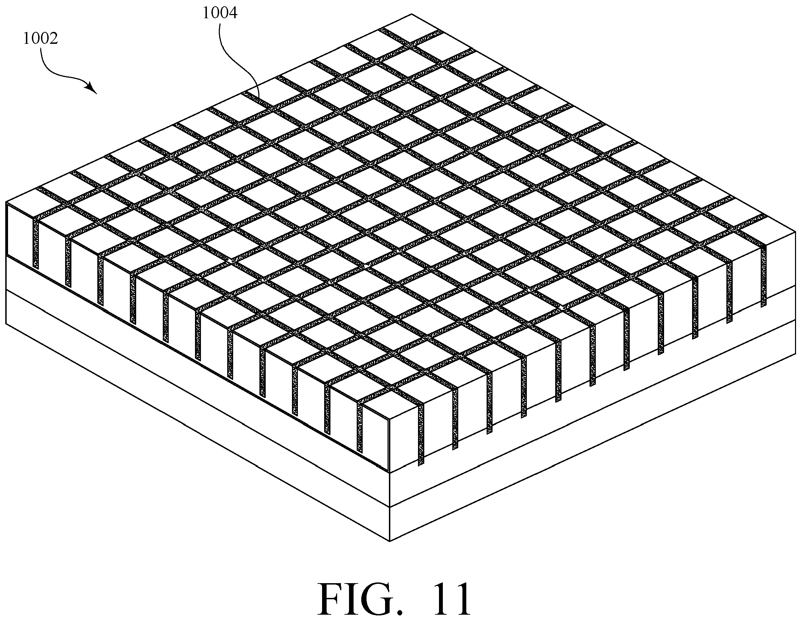

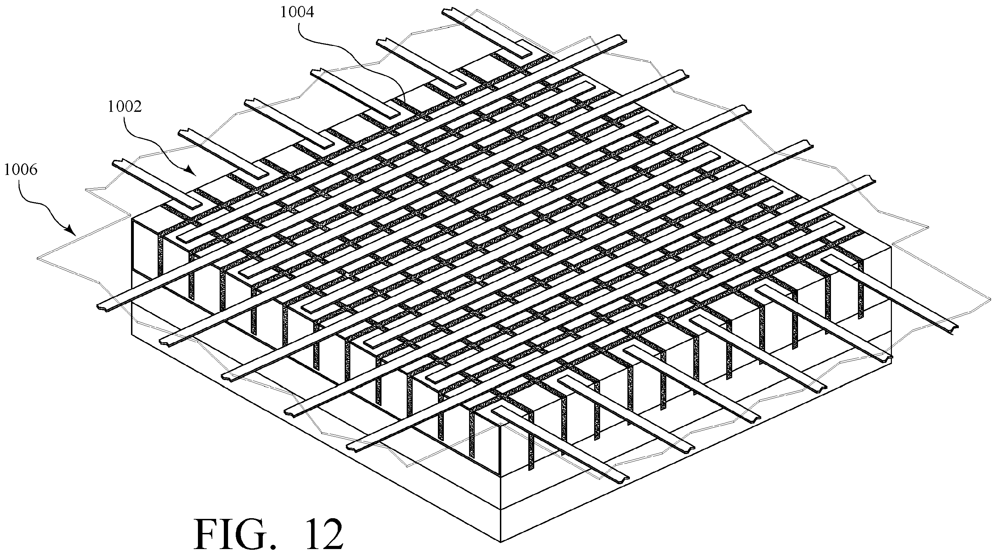

La figure 11 et la figure 12 illustrent une variante de mode de réalisation, similaire au mode de réalisation illustré en figure 7 et en figure 8, d'un ensemble intermédiaire après découpe d'une plaque de matériau actif métallisée 102 pour créer des traits de scie, dans lequel les traits de scie sont remplis avec, par exemple, un matériau polymère 1004 avant la fixation d'une couche d'interconnexion 1006. Le matériau polymère assure un découplage mécanique entre les éléments du transducteur et peut inclure des charges ou des bulles d'air.

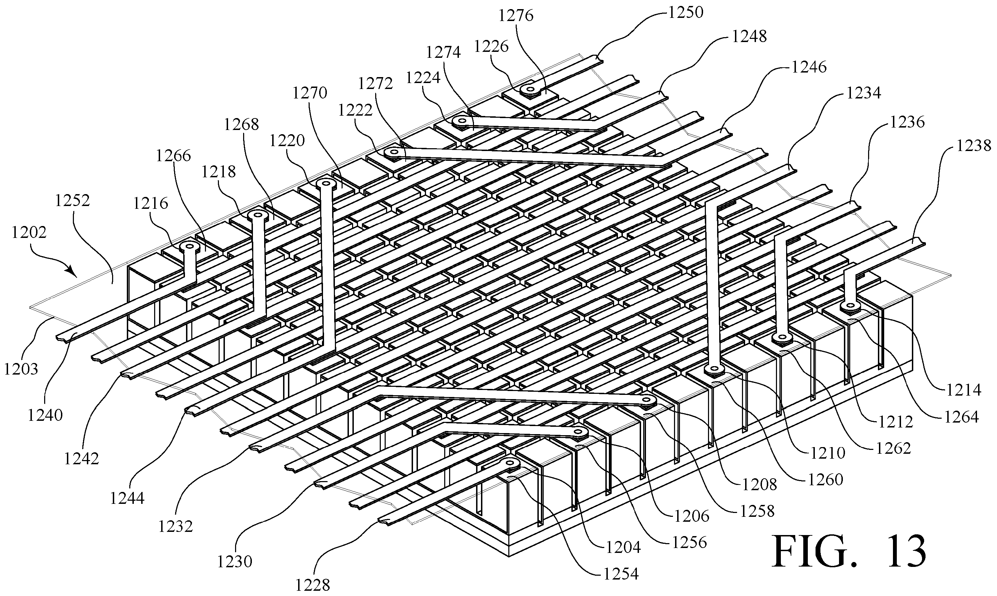

La figure 13 représente une autre variante de mode de réalisation, dans laquelle la couche d'interconnexion 1202 comporte un substrat isolant 1203 muni de distributions d'interconnexions sur les deux faces principales du substrat isolant 1203. La distribution d'interconnexions du deuxième ensemble de pistes conductrices 724, 726, 728, 730, 732, 734, 736, 738, 740, 742 de la couche d'interconnexion 702 est inchangée dans le cas de la couche d'interconnexions 1202. La couche d'interconnexion 1202 est munie d'un premier ensemble de pastilles de contact électrique 1204, 1206, 1208, 1210, 1212, 1214, situées sur la face principale interne du substrat isolant 1203, et en contact électrique respectif parmi une première ligne externe de contacts d'électrode de colonne 1254, 1256, 1258, 1260, 1262, 1264 ; et d'un deuxième ensemble de pastilles de contact électrique 1216, 1218, 1220, 1222, 1224, 1226 situé sur la face principale interne du substrat isolant 1203, et en contact électrique respectif parmi une deuxième ligne externe de contacts d'électrode de colonne 1266, 1268, 1270, 1272, 1274, 1276. Des vias, c'est-à-dire des connexions électriques verticales traversant le substrat isolant 1203, connectent respectivement un premier ensemble de pastilles de contact électrique 1204, 1206, 1208, 1210, 1212, 1214, à un premier ensemble de pistes conductrices 1228, 1230, 1232, 1234, 1236, 1238 ; et un deuxième ensemble de pastilles de contact électrique 1216, 1218, 1220, 1222, 1224, 1226 respectivement à un deuxième ensemble de de pistes conductrices 1240, 1242, 1244, 1246, 1248, 1250. Ce mode de réalisation est particulièrement avantageux en ce qu'il permet de doubler le nombre de pistes conductrices par unité de surface de la couche d'interconnexion 1202. L'ensemble des pistes d'interconnexion électrique 1234, 1236, 1236, 1246, 1248, 1250, forme une première distribution d'interconnexions électrique sur un première face latérale du dispositif de transduction RCA. De même, l'ensemble des pistes d'interconnexion électrique 1228, 1230, 1232, 1240, 1242, 1242, forme une seconde distribution d'interconnexions électrique sur un deuxième face latérale du dispositif de transduction RCA opposée à la première face latérale. Dans une variante de mise en œuvre, la première et la seconde distribution d'interconnexions électrique sortent par une même face latérale du dispositif de transduction RCA. Toutefois, on notera en outre que si les connexions électriques avec l'ensemble d'électrodes de colonne sont formées sur la même surface latérale de la plaque de matériau actif métallisée 302, et que les connexions électriques avec les lignes d'électrodes de ligne sont formées sur la même surface latérale de la plaque de matériau actif métallisée 302, alors le nombre de distributions peut être réduit à un en plaçant les pistes conductrices respectives sur des côtés opposés du substrat 1203 et en utilisant des vias et des pastilles de connexion.

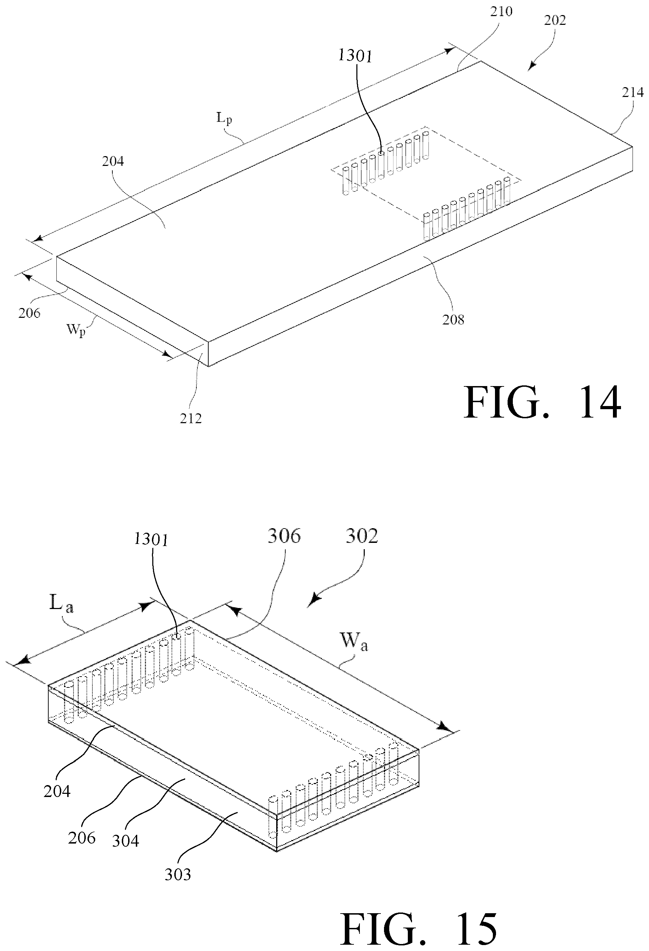

Les figures 14, 15 et 16 illustrent une variante de réalisation utilisant des vias métallisés 1301 traversant verticalement la plaque de matériau actif 102 et connectant électriquement les électrodes de colonne revêtant la face inférieure 106 de la plaque de matériau actif 102 (formées dans la couche métallique de face inférieure 206), à la couche métallique de face supérieure 204.

Les vias métallisés 1301 sont par exemple disposés aux extrémités des colonnes du transducteur ultrasonore. A titre d'exemple, chaque colonne comprend deux vias métallisés 1301 disposés respectivement aux deux extrémités de la colonne. A titre d'exemple, chaque colonne peut comprendre un unique via métallisé disposé à une extrémité de la colonne. A titre de variante, chaque colonne peut comprendre plus de deux vias métallisés 1301.

Dans cet exemple, les vias métallisés 1301 remplacent fonctionnellement les connecteurs d'électrode de colonne 518, 520, 522, 524, 526, 528, 530, 532, 534, et les connecteurs d'électrode de colonne 538, 540, 542, 544, 546, 548, 550, 552, 554, qui connectent électriquement les électrodes de colonne à la première couche métallique de surface principale 204.

Ainsi, dans cet exemple, les faces latérales de la plaque de matériau actif 102 aux extrémités des colonnes peuvent être non métallisées.

Le procédé de formation du transducteur est similaire à ce qui a été décrit précédemment en relation avec les figures 2 à 13. Seules les différences par rapport aux procédés des figures 2 à 13 sont détaillées ci-après.

Le procédé des figures 14, 15 et 16 diffère des exemples décrits en relation avec les figures 2 à 13 en ce que, dans l'exemple des figures 14, 15 et 16, des ouvertures traversantes, par exemple cylindriques, sont formées dans la plaque de matériau actif brute 102 aux emplacements souhaités des vias 1301 du futur transducteur ultrasonore RCA.

Les ouvertures sont par exemple formées par photolithographie et gravure, ou par toute autre méthode adaptée de formation d'ouvertures traversantes dans une plaque de matériau actif brute.

Une métallisation enveloppante est ensuite formée pour obtenir une plaque de matériau actif brute métallisée 202, de façon similaire à ce qui a été décrit précédemment en relation avec la figure 3. Lors de cette étape, les ouvertures traversantes sont remplies de métal ou ont leurs flancs revêtus de métal, formant les vias métallisés 1301.

La figure 14 est une vue en perspective de la plaque de matériau actif brute métallisée 202 obtenue à l'issue de cette étape. Sur cette figure, les vias métallisés 1301 sont représentés en transparence à titre d'illustration.

La plaque de matériau actif brute métallisée 202 est ensuite découpée pour individualiser une plaque de matériau actif métallisée 302 aux dimensions souhaitées du transducteur ultrasonore, de façon similaire à ce qui a été décrit ci-dessus en relation avec la figure 4.

La figure 15 est une vue en perspective illustrant de façon schématique la plaque de matériau actif métallisée 302 obtenue à l'issue de l'étape de découpe.

On notera que dans cet exemple, les dimensions de la plaque de matériau actif métallisée 302 peuvent être inférieures aux dimensions de la plaque de matériau actif brute métallisée 202. En particulier, la largeur Wa de la plaque de matériau actif métallisée 302 peut être inférieure à la largeur Wp de la plaque de matériau actif brute métallisée 202, et la longueur La de la plaque de matériau actif métallisée 302 peut être inférieure à la longueur Lp de la plaque de matériau actif brute métallisée 202. Dans ce cas, aucune des faces latérales de la plaque 302 n'est métallisée. Autrement dit, les surfaces latérales de la plaque de matériau actif 303 sont toutes exposées, comme cela est illustré en figure 15.

Les étapes suivantes du procédé sont par exemple identiques ou similaires à ce qui a été décrit précédemment en relation avec les figures 5, 6, 7, 8, 9, 10, et, éventuellement, 11, 12 et/ou 13.

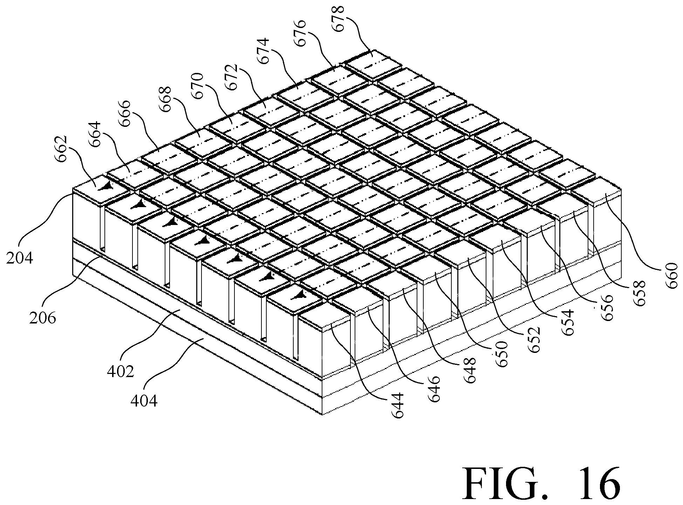

La figure 16 illustre la structure obtenue à un stade intermédiaire du procédé, correspondant à la structure obtenue à l'issue des étapes décrites en relation avec les figures 5, 6 et 7.

Un avantage du mode de réalisation décrit en relation avec les figures 14, 15 et 16 est qu'il permet de former des transducteurs dont les dimensions latérales sont inférieures à celles de la plaque brute, c'est à dire que plusieurs plaques actives peuvent être découpées dans les deux directions latérales. Dans ce cas, les côtés latéraux de la plaque active ne sont plus métallisés pour former un enveloppement tel que décrit dans les exemples des figures 2 à 13. La fonction de connexion électrique des électrodes de colonne situées du côté de la face inférieure de la plaque active à des métallisations situées du côté de la face supérieure de la plaque active est alors assurée par les vias métallisés 1301.

On comprendra que différents détails de l'objet décrit ici peuvent être modifiés sans s'écarter du cadre de l'objet décrit et revendiqué ici. En outre, la description précédente a pour seul objet d'illustrer et non de limiter. A titre d'exemple, les nombres de lignes et de colonnes des transducteurs ultrasonores en réseau (RCA) représentés ont été simplifiés afin d'illustrer, alors que les transducteurs ultrasonores réels peuvent comporter beaucoup plus d'éléments dans chaque direction, par exemple 64, 128, 256, ou plus d'éléments transducteur dans chacune des directions de lignes et de colonnes.

## Revendications

1. Dispositif de transduction ultrasonore matriciel à adressage ligne-colonne, comprenant :
- une plaque de matériau actif métallisée (302) comportant une plaque de matériau actif (303) ayant une première surface principale (104) et une deuxième surface principale (106) à l'opposé de la première surface principale, une première couche métallique de surface principale (204) sur la première surface principale (104), et une deuxième couche métallique de surface principale (206) sur la deuxième surface principale (106) ;
- une couche d'adaptation d'impédance acoustique (402, 404) fixée à la deuxième couche métallique de surface principale (206) ;
- un premier ensemble d'encoches de découpe (502, 504, 506, 508, 510, 512, 514, 516) parallèles orientées dans une première direction, lesdites encoches du premier ensemble s'étendant à travers toute l'épaisseur de la plaque de matériau actif métallisée (302) et sur toute la longueur de la plaque de matériau actif métallisée (302) dans la première direction, et individualisant la deuxième couche métallique de surface principale (206) en un ensemble d'électrodes de colonne ;
- un deuxième ensemble d'encoches de découpe (602, 604, 606, 608, 610, 612, 614, 616, 618, 620, 622) parallèles orientées selon une deuxième direction différente de la première direction, lesdites encoches du deuxième ensemble s'étendant à travers toute l'épaisseur de la première couche métallique de surface principale (204) et à travers au moins une partie de l'épaisseur de la plaque de matériau actif (303), et sur toute la longueur de la plaque de matériau actif métallisée (302) dans la deuxième direction, et individualisant la première couche métallique de surface principale (204) en un ensemble de lignes d'électrodes de ligne (624, 626, 628, 630, 632, 634, 636, 638, 640, 642) et une première ligne externe de contacts d'électrode de colonne (644, 646, 648, 650, 652, 654, 656, 658, 660), chaque contact d'électrode de colonne de la première ligne externe de contacts d'électrode de colonne étant connecté électriquement à une électrode de colonne respective de l'ensemble d'électrodes de colonne ; et
- une couche d'interconnexion (702) comportant un substrat (704) d'un matériau isolant électriquement, un premier ensemble de pistes conductrices (706, 710, 714, 716, 718, 722) sur le substrat (704), chaque piste du premier ensemble de pistes conductrices étant en contact électrique avec un contact d'électrode de colonne respectif (644, 648, 652, 656, 660) de la première ligne externe de contacts d'électrode de colonne, et un deuxième ensemble de pistes conductrices (724, 726, 728, 730, 732, 734, 736, 738, 740, 742) sur le substrat (704), chaque piste du deuxième ensemble de pistes conductrices étant en communication électrique avec une ligne respective parmi les lignes d'électrodes de ligne (624, 626, 628, 630, 632, 634, 636, 638, 640, 642).

2. Dispositif selon la revendication 1, dans lequel chaque contact d'électrode de colonne (644, 646, 648, 650, 652, 654, 656, 658, 660) de la première ligne externe de contacts d'électrode de colonne est connecté électriquement à l'électrode de colonne respective correspondante de l'ensemble d'électrodes de colonne par un connecteur d'électrode de colonne respectif d'un premier ensemble de connecteurs d'électrode de colonne (518, 520, 522, 524, 526, 528, 530, 532, 534) formé dans une première couche métallique de surface latérale (208) recouvrant une première surface latérale de la plaque de matériau actif (303), les connecteurs d'électrode de colonne du premier ensemble de connecteurs d'électrode de colonne étant individualisés par les encoches de découpe du premier ensemble d'encoches de découpe.

3. Dispositif selon la revendication 1, dans lequel chaque contact d'électrode de colonne (644, 646, 648, 650, 652, 654, 656, 658, 660) de la première ligne externe de contacts d'électrode de colonne est connecté électriquement à l'électrode de colonne respective correspondante de l'ensemble d'électrodes de colonne par un via métallisé (1301) traversant verticalement la plaque de matériau actif (303).

4. Dispositif selon l'une quelconque des revendications 1 à 3, dans lequel le substrat (704) de la couche d'interconnexion (702) comporte une surface interne, dans lequel le premier ensemble de pistes conductrices (706, 708, 710, 712, 714, 716, 718, 720, 722) et le deuxième ensemble de pistes conductrices (724, 726, 728, 730, 732, 734, 736, 738, 740, 742) sont sur la surface interne du substrat (704), et dans lequel le premier ensemble de pistes conductrices forme une première distribution d'interconnexions de la couche d'interconnexion, et le deuxième ensemble de pistes conductrices (724, 726, 728, 730, 732, 734, 736, 738, 740, 742) forme une deuxième distribution d'interconnexions de la couche d'interconnexion.

5. Dispositif selon l'une quelconque des revendications 1 à 3,
dans lequel le substrat (704) de la couche d'interconnexion (702) comporte une surface interne, une surface externe, des vias alignés respectivement avec la première ligne externe de contacts d'électrode de colonne, et des connecteurs de vias (1204, 1206, 1208, 1210, 1212, 1214, 1216, 1218, 1220, 1222, 1224, 1226) respectifs dans les vias ;
dans lequel le premier ensemble de pistes conductrices est sur la surface externe du substrat (704) et est en communication électrique avec des contacts d'électrode de colonne respectifs de la première ligne externe de contacts d'électrode de colonne au moyen de connecteurs de vias, et le deuxième ensemble de pistes conductrices est sur la surface interne du substrat (704) ; et
dans lequel le premier ensemble de pistes conductrices et le deuxième ensemble de pistes conductrices forment une première distribution d'interconnexions de la couche d'interconnexion.

6. Dispositif selon l'une quelconque des revendications 1 à 5,
dans lequel le deuxième ensemble d'encoches de découpe (602, 604, 606, 608, 610, 612, 614, 616, 618, 620, 622) parallèles individualise en outre une deuxième ligne externe de contacts d'électrode de colonne (662, 668, 670, 672, 674, 676, 678, 680), chaque contact d'électrode de colonne de la première ligne externe de contacts d'électrode de colonne étant connecté électriquement à une électrode de colonne respective de l'ensemble d'électrodes de colonne.

7. Dispositif selon l'une quelconque des revendications 1 à 6, dans lequel les première et deuxième directions sont orthogonales.

8. Dispositif selon l'une quelconque des revendications 1 à 7, dans lequel le premier ensemble d'encoches de découpe et le deuxième ensemble d'encoches de découpe sont remplis avec un matériau polymère (1004).

9. Dispositif selon l'une quelconque des revendications 1 à 8, dans lequel la couche d'interconnexion (702) est une carte de circuit imprimé flexible.

10. Dispositif selon l'une quelconque des revendications 1 à 9, comprenant en outre un ensemble de couches de face arrière (902) fixées à la couche d'interconnexion (702).

11. Procédé de fabrication d'un dispositif de transduction ultrasonore matriciel à adressage ligne-colonne, le procédé comprenant :
la fourniture d'une plaque brute de matériau actif métallisée (202) comportant une plaque de matériau actif (103) ayant une première surface principale (104) et une deuxième surface principale (106) à l'opposé de la première surface principale, une première couche métallique de surface principale (204) sur la première surface principale (104), et une deuxième couche métallique de surface principale (206) sur la deuxième surface principale (106),
la plaque brute de matériau actif métallisée ayant une largeur Wp et une longueur Lp ;
la découpe de la plaque brute de matériau actif métallisée (202) pour individualiser une plaque de matériau actif métallisée (302) à partir de la plaque brute de matériau actif métallisée (202) ;
la fixation d'une couche d'adaptation d'impédance acoustique à la deuxième couche métallique de surface principale (206) de la plaque de matériau actif métallisée (302) ;
la découpe complète de la plaque de matériau actif métallisée (302) dans une première direction pour former un premier ensemble d'encoches de découpe parallèles, le premier ensemble d'encoches de découpe formant dans la deuxième couche métallique de surface principale (206) un ensemble d'électrodes de colonne ;
la découpe partielle de la plaque de matériau actif métallisée (302) dans une deuxième direction pour former un deuxième ensemble d'encoches de découpe parallèles, le deuxième ensemble d'encoches de découpe formant dans la première couche métallique (204) de surface principale des lignes d'électrodes de ligne et une première ligne externe de contacts d'électrode de colonne, chaque contact d'électrode de colonne de la première ligne externe de contacts d'électrode de colonne étant connecté électriquement à une électrode de colonne respective de l'ensemble d'électrodes de colonne ;
la fourniture d'une couche d'interconnexion (702) comportant un substrat (704) d'un matériau électriquement isolant, un premier ensemble de pistes conductrices sur le substrat électriquement isolant (704), un deuxième ensemble de pistes conductrices sur le substrat électriquement isolant (704) ;
la connexion de chaque piste du premier ensemble de pistes conductrices pour qu'elle soit en communication électrique avec un contact d'électrode de colonne respectif de la première ligne externe de contacts d'électrode de colonne ; et
la connexion de chaque piste du deuxième ensemble de pistes conductrices en contact électrique avec une ligne respective parmi les lignes d'électrodes de ligne.

12. Procédé selon la revendication 11, dans lequel chaque contact d'électrode de colonne (644, 646, 648, 650, 652, 654, 656, 658, 660) de la première ligne externe de contacts d'électrode de colonne est connecté électriquement à l'électrode de colonne respective correspondante de l'ensemble d'électrodes de colonne par un connecteur d'électrode de colonne respectif d'un premier ensemble de connecteurs d'électrode de colonne (518, 520, 522, 524, 526, 528, 530, 532, 534) formé dans une première couche métallique de surface latérale (208) de la plaque de matériau actif métallisée (302), recouvrant une première surface latérale de la plaque de matériau actif (303), les connecteurs d'électrode de colonne du premier ensemble de connecteurs d'électrode de colonne étant individualisés par les encoches de découpe du premier ensemble d'encoches de découpe.

13. Procédé selon la revendication 12, dans lequel la plaque de matériau actif métallisée (302) a une longueur La inférieure à la longueur Lp de la plaque brute de matériau actif métallisée (202) et une largeur Wa égale à la largueur Wp de la plaque brute de matériau actif métallisée (202).

14. Procédé selon la revendication 11, dans lequel chaque contact d'électrode de colonne (644, 646, 648, 650, 652, 654, 656, 658, 660) de la première ligne externe de contacts d'électrode de colonne est connecté électriquement à l'électrode de colonne respective correspondante de l'ensemble d'électrodes de colonne par un via métallisé (1301) de la plaque de matériau actif métallisée (302), traversant verticalement la plaque de matériau actif (303).

15. Procédé selon la revendication 14, dans lequel la plaque de matériau actif métallisée (302) a une longueur La inférieure à la longueur Lp de la plaque brute de matériau actif métallisée (202) et une largeur Wa inférieure à la largueur Wp de la plaque brute de matériau actif métallisée (202).

## Patentansprüche

1. Ultraschall-Übertragungs-Vorrichtung mit Zeilen-Spalten-Adressierungs-Array, die Folgendes aufweist:
- eine metallisierte Platte aus aktivem Material (302) aufweisend eine Platte aus aktivem Material (303) mit einer ersten Hauptoberfläche (104) und einer zweiten Hauptoberfläche (106) gegenüber der ersten Hauptoberfläche, eine erste metallische Hauptoberflächenschicht (204) auf der ersten Hauptoberfläche (104) und eine zweite metallische Hauptoberflächenschicht (206) auf der zweiten Hauptoberfläche (106);
- eine akustische Impedanzanpassungsschicht (402, 404), gebondet mit der zweiten metallischen Hauptoberflächenschicht (206);
- einen ersten Satz von parallelen Schneidkerben (502, 504, 506, 508, 510, 512, 514, 516), die in einer ersten Richtung ausgerichtet sind, wobei sich die Kerben des ersten Satzes über die gesamte Dicke der metallisierten Platte aus aktivem Material (302) und entlang der gesamten Länge der metallisierten Platte aus aktivem Material (302) in der ersten Richtung erstrecken, und die zweite metallische Hauptoberflächenschicht (206) in einen Satz von Säulenelektroden individualisieren;
- einen zweiten Satz paralleler Schneidkerben (602, 604, 606, 608, 610, 612, 614, 616, 618, 620, 622), die entlang einer zweiten Richtung ausgerichtet sind, die sich von der ersten Richtung unterscheidet, wobei sich die Kerben des zweiten Satzes über die gesamte Dicke der ersten metallischen Hauptoberflächenschicht (204) und über wenigstens einen Teil der Dicke der Platte aus aktivem Material (303) und entlang der gesamten Länge der metallisierten Platte aus aktivem Material (302) in der zweiten Richtung erstrecken, und die erste metallische Hauptoberflächenschicht (204) in einen Satz von Zeilen von Zeilenelektroden (624, 626, 628, 630, 632, 634, 636, 638, 640, 642) und eine erste externe Zeile von Spaltenelektrodenkontakten (644, 646, 648, 650, 652, 654, 656, 658, 660) individualisiert, wobei jeder Spaltenelektrodenkontakt der ersten externen Zeile von Spaltenelektrodenkontakten elektrisch mit einer entsprechenden Spaltenelektrode des Satzes von Spaltenelektroden verbunden ist; und
- eine Verbindungsschicht (702) aufweisend ein Substrat (704) aus einem elektrisch isolierenden Material, einen ersten Satz von leitenden Pfaden (706, 710, 714, 716, 718, 722) auf dem Substrat (704), wobei jeder Pfad des ersten Satzes von leitenden Pfaden in elektrischem Kontakt mit einem entsprechenden Spaltenelektrodenkontakt (644, 648, 652, 656, 660) der ersten externen Zeile von Spaltenelektrodenkontakten steht, und einen zweiten Satz von leitenden Pfaden (724, 726, 728, 730, 732, 734, 736, 738, 740, 742) auf dem Substrat (704), wobei jeder Pfad des zweiten Satzes von leitenden Pfaden in elektrischer Verbindung mit einer entsprechenden Zeile aus den Zeilen von Zeilenelektroden (624, 626, 628, 630, 632, 634, 636, 638, 640, 642) steht.

2. Vorrichtung nach Anspruch 1, wobei jeder Spaltenelektrodenkontakt (644, 646, 648, 650, 652, 654, 656, 658, 660) der ersten externen Zeile von Spaltenelektrodenkontakten elektrisch verbunden ist mit der jeweiligen entsprechenden Spaltenelektrode des Satzes von Spaltenelektroden durch einen jeweiligen Spaltenelektrodenverbinder eines ersten Satzes von Spaltenelektrodenverbindern (518, 520, 522, 524, 526, 528, 530, 532, 534), die in einer ersten lateralen Oberflächenmetallschicht (208) ausgebildet sind, die eine erste laterale Oberfläche der Platte aus aktivem Material (303) bedeckt, wobei die Spaltenelektrodenverbinder des ersten Satzes von Spaltenelektrodenverbindern durch die Schneidkerben des ersten Satzes von Schneidkerben individualisiert sind.

3. Vorrichtung nach Anspruch 1, wobei jeder Spaltenelektrodenkontakt (644, 646, 648, 650, 652, 654, 656, 658, 660) der ersten externen Zeile von Spaltenelektrodenkontakten elektrisch verbunden ist mit der jeweiligen entsprechenden Spaltenelektrode des Satzes von Spaltenelektroden durch ein metallisiertes Via (1301), das die Platte aus aktivem Material (303) vertikal durchquert.

4. Vorrichtung nach einem der Ansprüche 1 bis 3, wobei das Substrat (704) der Verbindungsschicht (702) eine innere Oberfläche aufweist, wobei der erste Satz von leitenden Pfaden (706, 708, 710, 712, 714, 716, 718, 720, 722) und der zweite Satz von leitenden Pfaden (724, 726, 728, 730, 732, 734, 736, 738, 740, 742) auf der inneren Oberfläche des Substrats (704) sind, und wobei der erste Satz von leitenden Pfaden einen ersten Verbindungsfächer der Verbindungsschicht ausbildet, und der zweite Satz von leitenden Pfaden (724, 726, 728, 730, 732, 734, 736, 738, 740, 742) einen zweiten Verbindungsfächer der Verbindungsschicht ausbildet.

5. Vorrichtung nach einem der Ansprüche 1 bis 3,
wobei das Substrat (704) der Verbindungsschicht (702) eine innere Oberfläche aufweist, eine äußere Oberfläche, Vias, die jeweils mit der ersten externen Zeile von Spaltenelektrodenkontakten ausgerichtet sind, und entsprechende Via-Verbinder (1204, 1206, 1208, 1210, 1212, 1214, 1216, 1218, 1220, 1222, 1224, 1226) in den Vias;
wobei sich der erste Satz von leitenden Pfaden auf der äußeren Oberfläche des Substrats (704) befindet und in elektrischer Verbindung mit entsprechenden Spaltenelektrodenkontakten der ersten externen Zeile von Spaltenelektrodenkontakten mittels Via-Verbindern steht, und der zweite Satz von leitenden Pfaden sich auf der inneren Oberfläche des Substrats (704) befindet; und
wobei der erste Satz von leitenden Pfaden und der zweite Satz von leitenden Pfaden einen ersten Verbindungsfächer der Verbindungsschicht ausbilden.

6. Vorrichtung nach einem der Ansprüche 1 bis 5,
wobei der zweite Satz paralleler Schneidkerben (602, 604, 606, 608, 610, 612, 614, 616, 618, 620, 622) ferner eine zweite externe Zeile von Spaltenelektrodenkontakten (662, 668, 670, 672, 674, 676, 678, 680) individualisiert, wobei jeder Spaltenelektrodenkontakt der ersten externen Zeile von Spaltenelektrodenkontakten elektrisch verbunden mit einer jeweiligen Spaltenelektrode des Satzes von Spaltenelektroden ist.

7. Vorrichtung nach einem der Ansprüche 1 bis 6, wobei die erste und die zweite Richtung orthogonal zueinander sind.

8. Vorrichtung nach einem der Ansprüche 1 bis 7, wobei der erste Satz von Schneidkerben und der zweite Satz von Schneidkerben mit einem Polymermaterial (1004) gefüllt sind.

9. Vorrichtung nach einem der Ansprüche 1 bis 8, wobei die Verbindungsschicht (702) eine gedruckte Flex-Leiterplatte ist.

10. Vorrichtung nach einem der Ansprüche 1 bis 9, die ferner einen Satz von Stützschichten (902) aufweist, die mit der Verbindungsschicht (702) verbunden sind.

11. Verfahren zum Herstellen einer Ultraschall-Übertragungs-Vorrichtung mit Zeilen-Spalten-Adressierungs-Array, wobei das Verfahren Folgendes aufweist:
das Bereitstellen einer metallisierten Rohplatte aus aktivem Material (202) aufweisend eine Platte aus aktivem Material (103) mit einer ersten Hauptoberfläche (104) und einer zweiten Hauptoberfläche (106) gegenüber der ersten Hauptoberfläche, eine erste metallische Hauptoberflächenschicht (204) auf der ersten Hauptoberfläche (104) und eine zweite metallische Hauptoberflächenschicht (206) auf der zweiten Hauptoberfläche (106), wobei die metallisierte Rohplatte aus aktivem Material eine Breite Wp und eine Länge Lp hat;
das Schneiden der metallisierten Rohplatte aus aktivem Material (202) zum Individualisieren einer metallisierten Platte aus aktivem Material (302) aus der metallisierten Rohplatte aus aktivem Material (202);
das Bonden einer akustischen Impedanzanpassungsschicht auf die zweite metallische Hauptoberflächenschicht (206) der metallisierten Platte aus aktivem Material (302);
das vollständige Schneiden der metallisierten Platte aus aktivem Material (302) in einer ersten Richtung zum Ausbilden eines ersten Satzes von parallelen Schneidkerben, wobei der erste Satz von Schneidkerben in der zweiten metallischen Hauptoberflächenschicht (206) einen Satz von Spaltenelektroden ausbildet;
das teilweise Schneiden der metallisierten Platte aus aktivem Material (302) in einer zweiten Richtung zum Ausbilden eines zweiten Satzes von parallelen Schneidkerben, wobei der zweite Satz von Schneidkerben in der ersten metallischen Hauptoberflächenschicht (204) Zeilen von Zeilenelektroden und eine erste externe Zeile von Spaltenelektrodenkontakten ausbildet, wobei jeder Spaltenelektrodenkontakt der ersten externen Zeile von
Spaltenelektrodenkontakten elektrisch verbunden mit einer entsprechenden Spaltenelektrode des Satzes von Spaltenelektroden ist;
das Bereitstellen einer Verbindungsschicht (702) aufweisend ein Substrat (704) aus einem elektrisch isolierenden Material, einen ersten Satz von leitenden Pfaden auf dem elektrisch isolierenden Substrat (704), einen zweiten Satz von leitenden Pfaden auf dem elektrisch isolierenden Substrat (704);
das Verbinden jedes Pfades des ersten Satzes von leitenden Pfaden, so dass er elektrisch in Verbindung mit einem entsprechenden Spaltenelektrodenkontakt der ersten externen Zeile von Spaltenelektrodenkontakten steht; und
das Verbinden jedes Pfades des zweiten Satzes von leitenden Pfaden in elektrischem Kontakt mit einer entsprechenden Zeile der Zeilen von Zeilenelektroden.

12. Verfahren nach Anspruch 11, wobei jeder Spaltenelektrodenkontakt (644, 646, 648, 650, 652, 654, 656, 658, 660) der ersten externen Zeile von Spaltenelektrodenkontakten elektrisch verbunden ist mit der jeweiligen entsprechenden Spaltenelektrode des Satzes von Spaltenelektroden durch einen jeweiligen Spaltenelektrodenverbinder eines ersten Satzes von Spaltenelektrodenverbindern (518, 520, 522, 524, 526, 528, 530, 532, 534), die in einer ersten lateralen Oberflächenmetallschicht (208) der metallisierten Platte aus aktivem Material (302) ausgebildet sind, die eine erste laterale Oberfläche der Platte aus aktivem Material (303) bedeckt, wobei die Spaltenelektrodenverbinder des ersten Satzes von Spaltenelektrodenverbindern durch die Schneidkerben des ersten Satzes von Schneidkerben individualisiert sind.

13. Verfahren nach Anspruch 12, wobei die metallisierte Platte aus aktivem Material (302) eine Länge La kleiner als die Länge Lp der metallisierten Rohplatte aus aktivem Material (202) und eine Breite Wa gleich der Breite Wp der metallisierten Rohplatte aus aktivem Material (202) hat.

14. Verfahren nach Anspruch 11, wobei jeder Spaltenelektrodenkontakt (644, 646, 648, 650, 652, 654, 656, 658, 660) der ersten externen Zeile von Spaltenelektrodenkontakten elektrisch verbunden ist mit der jeweiligen entsprechenden Spaltenelektrode des Satzes von Spaltenelektroden durch ein metallisiertes Via (1301) der metallisierten Platte aus aktivem Material (302), das die Platte aus aktivem Material (303) vertikal durchquert.

15. Verfahren nach Anspruch 14, wobei die metallisierte Platte aus aktivem Material (302) eine Länge La hat, die kleiner ist als die Länge Lp der metallisierten Rohplatte aus aktivem Material (202), und eine Breite Wa, die kleiner ist als die Breite Wp der metallisierten Rohplatte aus aktivem Material (202).

## Claims

1. Row-column addressing array ultrasound transduction device, comprising:
- a metallized plate of active material (302) comprising a plate of active material (303) having a first main surface (104) and a second main surface (106) opposite to the first main surface, a first metallic main surface layer (204) on the first main surface (104), and a second metallic main surface layer (206) on the second main surface (106);
- an acoustic impedance matching layer (402, 404) bonded to the second metallic main surface layer (206);
- a first set of parallel cutting notches (502, 504, 506, 508, 510, 512, 514, 516) oriented in a first direction, said notches of the first set extending across the entire thickness of the metallized plate of active material (302) and along the entire length of the metallized plate of active material (302) in the first direction, and individualizing the second metallic main surface layer (206) into a set of column electrodes;
- a second set of parallel cutting notches (602, 604, 606, 608, 610, 612, 614, 616, 618, 620, 622) oriented along a second direction different from the first direction, said notches of second set extending across the entire thickness of the first metallic main surface layer (204) and across at least part of the thickness of the plate of active material (303), and along the entire length of the metallized plate of active material (302) in the second direction, and individualizing the first metallic main surface layer (204) into a set of rows of row electrodes (624, 626, 628, 630, 632, 634, 636, 638, 640, 642) and a first external row of column electrode contacts (644, 646, 648, 650, 652, 654, 656, 658, 660), each column electrode contact of the first external row of column electrode contacts being electrically connected to a respective column electrode of the set of column electrodes; and
- an interconnection layer (702) comprising a substrate (704) of an electrically-insulating material, a first set of conductive tracks (706, 710, 714, 716, 718, 722) on the substrate (704), each track of the first set of conductive tracks being in electrical contact with a respective column electrode contact (644, 648, 652, 656, 660) of the first external row of column electrode contacts, and a second set of conductive tracks (724, 726, 728, 730, 732, 734, 736, 738, 740, 742) on the substrate (704), each track of the second set of conductive tracks being in electrical communication with a respective row among the rows of row electrodes (624, 626, 628, 630, 632, 634, 636, 638, 640, 642) .

2. Device according to claim 1, wherein each column electrode contact (644, 646, 648, 650, 652, 654, 656, 658, 660) of the first external row of column electrode contacts is electrically connected to the respective corresponding column electrode of the set of column electrodes by a respective column electrode connector of a first set of column electrode connectors (518, 520, 522, 524, 526, 528, 530, 532, 534) formed in a first lateral surface metal layer (208) covering a first lateral surface of the plate of active material (303), the column electrode connectors of the first set of column electrode connectors being individualized by the cutting notches of the first set of cutting notches.

3. Device according to claim 1, wherein each column electrode contact (644, 646, 648, 650, 652, 654, 656, 658, 660) of the first external row of column electrode contacts is electrically connected to the respective corresponding column electrode of the set of column electrodes by a metallized via (1301) vertically crossing the plate of active material (303).

4. Device according to any of claims 1 to 3, wherein the substrate (704) of the interconnection layer (702) comprises an inner surface, wherein the first set of conductive tracks (706, 708, 710, 712, 714, 716, 718, 720, 722) and the second set of conductive tracks (724, 726, 728, 730, 732, 734, 736, 738, 740, 742) are on the inner surface of the substrate (704), and wherein the first set of conductive tracks forms a first interconnect fan-out of the interconnection layer, and the second set of conductive tracks (724, 726, 728, 730, 732, 734, 736, 738, 740, 742) forms a second interconnect fan-out of the interconnection layer.

5. Device according to any of claims 1 to 3,
wherein the substrate (704) of the interconnection layer (702) comprises an inner surface, an outer surface, vias respectively aligned with the first external row of column electrode contacts, and respective via connectors (1204, 1206, 1208, 1210, 1212, 1214, 1216, 1218, 1220, 1222, 1224, 1226) in the vias;
wherein the first set of conductive tracks is on the outer surface of the substrate (704) and is in electrical communication with respective column electrode contacts of the first external row of column electrode contacts by means of via connectors, and the second set of conductive tracks is on the inner surface of the substrate (704); and wherein the first set of conductive tracks and the second set of conductive tracks form a first interconnect fan-out of the interconnection layer.

6. Device according to any of claims 1 to 5,
wherein the second set of parallel cutting notches (602, 604, 606, 608, 610, 612, 614, 616, 618, 620, 622) further individualizes a second external row of column electrode contacts (662, 668, 670, 672, 674, 676, 678, 680), each column electrode contact of the first external row of column electrode contacts being electrically connected to a respective column electrode of the set of column electrodes.

7. Device according to any of claims 1 to 6, wherein the first and second directions are orthogonal.

8. Device according to any of claims 1 to 7, wherein the first set of cutting notches and the second set of cutting notches are filled with a polymer material (1004).

9. Device according to any of claims 1 to 8, wherein the interconnection layer (702) is a flex printed circuit board.

10. Device according to any of claims 1 to 9, further comprising a set of backing layers (902) bonded to the interconnection layer (702).

11. Method of manufacturing a row-column addressing array ultrasound transduction device, the method comprising:
the provision of a metallized raw plate of active material (202) comprising a plate of active material (103) having a first main surface (104) and a second main surface (106) opposite to the first main surface, a first metallic main surface layer (204) on the first main surface (104), and a second metallic main surface layer (206) on the second main surface (106), the metallized raw plate of active material having a width Wp and a length Lp;
the cutting of the metallized raw plate of active material (202) to individualize a metallized plate of active material (302) from the metallized raw plate of active material (202);
the bonding of an acoustic impedance matching layer to the second metallic main surface layer (206) of the metallized plate of active material (302);
the full cutting of the metallized plate of active material (302) in a first direction to form a first set of parallel cutting notches, the first set of cutting notches forming in the second metallic main surface layer (206) a set of column electrodes;
the partial cutting of the metallized plate of active material (302) in a second direction to form a second set of parallel cutting notches, the second set of cutting notches forming in the first metallic main surface layer (204) rows of row electrodes and a first external row of column electrode contacts, each column electrode contact of the first external row of column electrode contacts being electrically connected to a respective column electrode of the set of column electrodes;
the provision of an interconnection layer (702) comprising a substrate (704) of an electrically-insulating material, a first set of conductive tracks on the electrically-insulating substrate (704), a second set of conductive tracks on the electrically-insulating substrate (704);
the connection of each track of the first set of conductive tracks so that it is in electrical communication with a respective column electrode contact of the first external row of column electrode contacts; and
the connection of each track of the second set of conductive tracks in electrical contact with a respective row among the rows of row electrodes.

12. Method according to claim 11, wherein each column electrode contact (644, 646, 648, 650, 652, 654, 656, 658, 660) of the first external row of column electrode contacts is electrically connected to the respective corresponding column electrode of the set of column electrodes by a respective column electrode connector of a first set of column electrode connectors (518, 520, 522, 524, 526, 528, 530, 532, 534) formed in a first lateral surface metal layer (208) of the metallized plate of active material (302), covering a first lateral surface of the plate of active material (303), the column electrode connectors of the first set of column electrode connectors being individualized by the cutting notches of the first set of cutting notches.

13. Method according to claim 12, wherein the metallized plate of active material (302) has a length La smaller than the length Lp of the metallized raw plate of active material (202) and a width Wa equal to the width Wp of the metallized raw plate of active material (202).

14. Method according to claim 11, wherein each column electrode contact (644, 646, 648, 650, 652, 654, 656, 658, 660) of the first external row of column electrode contacts is electrically connected to the respective corresponding column electrode of the set of column electrodes by a metallized via (1301) of the metallized plate of active material (302), vertically crossing the plate of active material (303).

15. Method according to claim 14, wherein the metallized plate of active material (302) has a length La smaller than the length Lp of the metallized raw plate of active material (202) and a width Wa smaller than the width Wp of the metallized raw plate of active material (202)
